# EUROPEAN PATENT APPLICATION

(11) **EP 2 801 569 A1**
(43) Date of publication of application: **12.11.2014**
(21) Application number: 13382170.2
(22) Date of filing: 09.05.2013
(51) Int. Cl.: C07D 219/10, A61K 31/435, C07D 221/06, C07D 471/04, A61P 19/00

(54) **Histone deacetylase inhibitors based on derivatives of tricyclic polyhydroacridine and analogs possessing fused saturated five- and seven-membered rings**

(71) Applicant: Ikerchem, S.L., 20009 San Sebastian - Guipuzcoa (ES); Universidad del Pais Vasco, 48940 Leioa Vizcaya (ES)
(72) Inventor: Cossio Mora, Fernando Pedro, E-20018 San Sebastián - Guipúzcoa (ES); Vara Salazar, Yosu Ion, E-20009 San Sebastián - Guipúzcoa (ES); Masdeu Margalef, María del Carmen, E-20009 San Sebastián - Guipúzcoa (ES); Alcalà Caffarena, María Remedios, E-20009 San Sebastián - Guipúzcoa (ES); Villafruela Cáneva, Sergio, E-20009 San Sebastián - Guipúzcoa (ES); Otaegui Ansa, Dorleta, E-20009 San Sebastián - Guipúzcoa (ES); Aldaba Arévalo, Eneko, E-20009 San Sebastián - Guipúzcoa (ES); Zubia Olascoaga, Aizpea, E-20009 San Sebastián - Guipúzcoa (ES); San Sebastián Larzabal, Eider, E-20100 Rentería - Guipúzcoa (ES)
(74) Representative: ABG Patentes, S.L.

(57) **Abstract**

The present invention refers to compounds of formula (I): 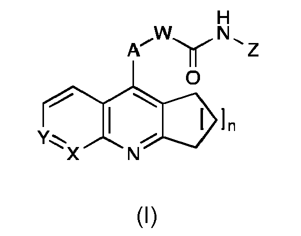 as well as to a method for their preparation, pharmaceutical compositions comprising the same, and use thereof for the treatment and/or chemoprevention of cancer hematological malignancy, proliferative diseases, neurological disorders and immunological disorders.

## Description

### FIELD OF THE INVENTION

The present invention is related to new compounds derived from tricyclic 1,2,3,4-tetrahydroacridine, 2,3-dihydro-1*H*-cyclopenta[b]quinoline, 7,8,9,10-tetrahydro-6*H-*cyclohepta[*b*]quinoline, 6,7,8,9-tetrahydrobenzo[*b*][1,8]naphthyridine and 6,7,8,9-tetrahydrobenzo[*b*][1,7]naphthyridine for their use as inhibitors of histone deacetylases and therapeutic agents for preventing or treating malignancies associated with aberrant histone acetylation such as cancer, hematological malignancies, proliferative diseases, neurological disorders, and immunological disorders.

### BACKGROUND OF THE INVENTION

Histone deacetylases (HDACs) are enzymes that, as part of multiprotein complexes or aggregates, catalyze the removal of acetyl groups from ε-NHAc lysine residues of histones and other proteins. These enzymes have been classified into three distinct structural classes. Classes I, II and IV HDACs are zinc-dependent whereas Class III HDACs use NAD as a cofactor. The biological functions of these classes of enzymes and the different isoforms belonging to each class are known. Class I HDACs include HDAC 1, 2, 3, 4 and 8 and are related, among other functions, with proliferation, gene regulation, apoptosis, morphogenesis and telomerase activity. Class IIa HDACs include HDAC 4, 5 and 7 and involve cardiac development, suppression of cardiac stress and regulation of apopotosis. HDAC 6 is included in Class IIb and it has been associated with the status of tubulin and hsp90 acetylation (cf. W. Sippl and M. Jung, Eds. Epigenetic Targets in Drug Discovery, Wiley-VCH: Weinheim, 2009). More recently, HDACs and their inhibitors have been associated with cell pluripotency, differentiation and reprogramming (cf. A. Kretsovali et al. Stem Cells International, 2012**,** Article ID 184154; doi: 10.1155/2012/184154).

The biological functions of HDACs have an extraordinary impact in diseases like cancer (L. Ellis and R. Pili, Parmaceuticals 2010, 3, 2441; L. Stimson et al. Annals of Oncology 2009, 20, 1293), central nervous system disorders (cf. A. G. Kazantsev and L. M. Thomson, Nat. Rev. Drug Discov. 2008, 7, 854), inflammation and immunity (cf. M. R. Shakespear et al. Trends in Immunology 2011, 32, 335) and HIV-1 latency (cf. N. M. Archin et al. Nature 2012, 487, 482).

Within this context, HDAC inhibitors (HDACi) have emerged as very relevant and promising drugs for the treatment of cancer (cf. R. W. Johnstone, Nat. Rev. Drug. Discov. 2002, 1, 287; M. Dokmanovic et al. Mol. Cancer Res. 2007, 5, 981; S. Ropero and M. Esteller, Mol. Oncology 2007, 1, 19). Although many of the actual mechanisms of anticancer activity of HDACi are not completely understood, there is evidence that these compounds alter the biological machinery affecting the hallmarks of cancer like apoptosis (HDAC 1 and 2) differentiation (HDAC 3, 4, 5 and 8), angiogenesis (HDAC 4, 6, 7 and 10), migration (HDAC 6), resistance to chemotherapy (HDAC 1) and proliferation (HDAC 1, 2, 3 and 8) (cf. O. Witt et al. Cancer Lett. 2009, 277, 8).

Suberoylanilide hydroxamic acid (SAHA) was the first HDACi approved in 2006 by the US FDA for the treatment of cutaneous T-cell lymphoma (CTCL) (cf. P. A. Marks, Oncogene 2007, 26, 1351; P. A. Marks, R. Breslow, Nat. Biotechnol. 2007, 25, 84). In 2009, disulfide FK228 gained approval by the same American agency (cf. C. Grant et al. Expert Rev. Anticancer Ther. 2010, 10, 997; E. M. Bertino et al. Expert Opin. Investig. Drugs 2011, 20, 1151). Nowadays, many HDACi have been prepared, some of them being in clinical development (cf. T. A. Miller et al. J. Med. Chem. 2003, 46, 5097; M. Paris et al. J. Med. Chem. 2008, 51, 1505). The current clinical status of these inhibitors both as monotherapy and in combined therapies (cf. S. T. Wong, Am. J. Health-Syst. Pharm. 2009, 66, S9) as well as the patents describing their main features has been reviewed (cf. F. Thaler, Pharm. Pat. Analyst 2012, 1, 75; M. L. Curtin, Expert Opin. Ther. Patents 2003, 12, 1375; T. A. Miller, Expert Opin. Ther. Patents 2004, 14, 791; H. Weinmann and E. Ottow, Expert Opin. Ther. Patents 2005, 15, 1677; S. Price and H. J. Dyke, Expert Opin. Ther. Patents 2007, 17, 745; H. Wang and B. W. Dymock, Expert Opin. Ther. Patents 2009, 19, 1727).

In general, HDACi possess common structural features like a relatively bulky capping group -that is intended to lie at the rim of the tunnel that connects the environment of the enzyme with the active site- an spacer -that mimics the side chain of the lysine residue and occupies the tunnel- and a chelating group that binds the Zn (II) metallic center of the active site (cf. A. Villar-Garea and M. Esteller, Int. J. Cancer 2004, 112, 171). Very recently HDACi that lack this latter chelating group have been described (cf. C. J. Vickers et al. ACS Med. Chem. Lett. 2012, 3, 505).

The chelating metal-binding moieties usually present in HDACi are hydroxamic acids and other groups like thiols and disulfides (the latter probably giving rise in vivo to the corresponding thiolates), epoxides and benzamides (cf. T. Suzuki and N. Miyata, Curr. Med. Chem. 2005, 12, 2867)

Among the capping groups, cyclic peptides and related derivatives, 1*H*-indoles (cf. Y. Dai et al. Bioorg. Med. Chem. Lett. 2003, 13, 1897), 1*H*-pyrroles (cf. A. Zubia et al. Oncogene 2008, 28, 1477), aromatic groups (cf. E. Pontiki and D. Hadjipavlou-Litina, Med. Res. Rev. 2011, 32, 165) and tricyclic systems like dibenzo[*b,f*][1,4]thiazepin-11(10*H*)-ones and related *O*- and *N*- analogs (cf. M. Blnaschi et al. ACS Med. Chem. Lett. 2010, 1, 411) have been described as convenient components of HDACi.

Although different combinations of capping groups, spacers and chelating moieties have been prepared and tested, the most convenient combination of these building blocks is unknown. Thus, the HDACi activity of a new molecule combining these components cannot be predicted a priori, both in terms of potency and selectivity among the different HDAC isoforms. Actually, most of the HDACi reported so far are pan-inhibitors, which can generate undesired side effects (cf. A. V. Bieliauskas and M. K. H. Pflum, Chem. Soc. Rev. 2008, 37, 1402).

1,2,3,4-Tetrahydroacridin-9-amine *(Tacrine)* is a centrally acting cholinesterase inhibitor in use for the treatment of Alzheimer's disease. Other acridins have been prepared and tested within the same therapeutic field (cf. V. Tumiatti et al. Curr. Med. Chem. 2010, 17, 1825). Acridin (cf. L.Gupta, M. S. Chauhan, Chem. & Biol. Interface, 2011, 1,1,1-43) or tetrahydroacridin derivatives reported in the field of oncology are scarce. Thus, several amidoacridine derivatives have been described as selective inhibitors of ubiquitin specific protease 7 (cf. R. Lopez and F. Coland, *Eur. Pat. Appl.* **2011,** EP2357176 A1, WO 2011/986178 A1). Likewise, a number of acridine and quinoline derivatives have been reported as sirtuin modulators (cf. M. Milburn et al. PCT Int. Appl. 2006 WO 2006094237 A1, US 2009/0069301 A1).

However, to the best of our knowledge, the activity of tetrahydroacridine derivatives and related compounds as HDACi has not been reported. In particular, the interaction between these heterocycles with different spacers and chelating groups in order to generate HDACi activity is unknown.

### OBJECT OF THE INVENTION

A first aspect of the invention refers to compounds of general formula (I), 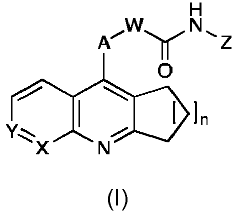 wherein:
X and Y are independently selected from a N atom or a C-R group, wherein R is selected from a hydrogen atom, an alkyl, an alkoxyl group and a hydroxyl group;
n is an integer selected from 1, 2 and 3;
A is a -NH- group or a -C(O)NH- group;
W represents a spacer group selected from -(CH₂)ₘ-, where m is 5 or 6, and the group of formula (II): 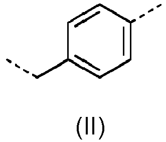
wherein the dashed lines represent the covalent unions with the groups A and -C(=O)-NH-Z;
Z is selected from a hydroxyl group and a group of formula (III): 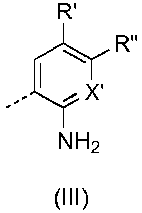 wherein:
   the dashed line represents the covalent union with group W-C(=O)-NH-;
   X' is selected from a -CH- group and a N atom; and
   R' and R" are independently selected from a H atom, an alkyl, a substituted or unsubstituted aryl and a heteroaryl group;
or a solvate or a salt or prodrug thereof.

Likewise, another aspect of the invention refers to the process for the preparation of compounds of general formula (I), or a solvate or a salt or prodrug thereof.

Another aspect of the present invention relates to a compound of general formula (I), or a salt, solvate or prodrug thereof, for its use as a medicament.

Another aspect of the present invention relates to a compound of general formula (I), or a salt, solvate or prodrug thereof, for its use in the treatment of cancer, hematological malignancy, proliferative diseases, neurological disorders and immunological disorders.

Another aspect of the present invention relates to the use of a compound of general formula (I), or a salt, solvate or prodrug thereof, in the preparation of a medicament for the treatment of cancer, hematological malignancy and proliferative diseases, proliferative diseases, neurological disorders and immunological disorders.

According to another aspect, the present invention is directed to a method of treating cancer, hematological malignancy, proliferative diseases, neurological disorders and immunological disorders, which comprises the administration to a patient needing such treatment, of a therapeutically effective amount of at least one compound of general formula (I) or a salt, solvate or prodrug thereof.

A further object of the invention is a pharmaceutical composition comprising at least one compound of general formula (I), or a salt, solvate or prodrug thereof, and at least one pharmaceutically acceptable excipient.

### DETAILED DESCRIPTION OF THE INVENTION

First, the present invention provides compounds of general formula (I), 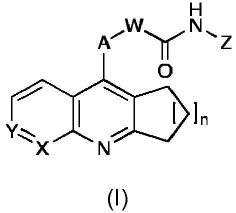 wherein:
X and Y are independently selected from a N atom or a C-R group, wherein R is selected from a hydrogen atom, an alkyl, an alkoxy group and a hydroxy group;
n is an integer selected from 1, 2 and 3;
A is a -NH- group or a -C(O)NH- group;
W represents a spacer group selected from -(CH₂)ₘ-, where m is 5 or 6, and the group of formula (II): 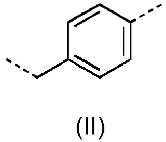
wherein the dashed lines represent the covalent unions with the groups A and -C(=O)-NH-Z;
Z is selected from a hydroxy group and a group of formula (III): 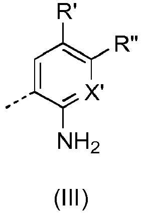 wherein:
   the dashed line represents the covalent union with group W-C(=O)-NH-;
   X' is selected from a -CH- group and a N atom; and
   R' and R" are independently selected from a H atom, an alkyl, a substitued or unsubstituted aryl and a heteroaryl group;
or a solvate or a salt or prodrug thereof.

The term "alkyl" refers to a linear or branched hydrocarbon chain radical consisting of carbon and hydrogen atoms, containing no unsaturation, having 1 to 6 carbon atoms, which is attached to the rest of the molecule by a single bond. Exemplary alkyl groups can be methyl, ethyl, n-propyl, i-propyl, n-butyl, i-butyl or *tert-*butyl.

The term "alkoxyl" refers to a radical of the formula -O-alkyl, wherein "alkyl" is as defined above. In an embodiment of the invention, alkoxyl refers to a radical of formula -O-C₁-C₃ alkyl. Exemplary alkoxyl radicals are methoxyl, ethoxyl, n-propoxyl or i-propoxyl.

The term "aryl" refers to a C₆-C₁₀ aromatic group comprising 1, 2 or 3 aromatic rings, linked by a carbon-carbon bond or condensed, optionally substituted with a group selected from a C₁-C₆ alkyl, halogen, nitro, cyano, OR^{a}, SR^{a}, SOR^{a}, SO₂R^{a}, NR^{a}R^{b}, C(O)R^{a}, C(O)OR^{a}, C(O)NR^{a}R^{b} or OC(O)R^{a}, wherein R^{a} and R^{b} are hydrogen or an alkyl group as defined above. The term aryl includes for example, and in a nonlimiting sense, phenyl, naphthyl, biphenyl, indenyl, etc.

The term "heteroaryl" refers to a stable 3 to 10 membered aromatic ring, preferably a 5 or 6 membered aromatic ring, which consists of carbon atoms and from one to five heteroatoms selected from nitrogen, oxygen and sulfur. For the purposes of this invention, the heteroaryl radical may be a monocyclic, bicyclic or tricyclic ring system, which may include condensed ring systems; and nitrogen, carbon or sulfur atoms in the heteroaryl radical may be optionally oxidized; and the nitrogen atom may be optionally quaternized. Examples of such heteroaryl include, but are not limited to, benzimidazole, benzothiazole, furan, thiophene, pyrrole, pyridine, pyrimidine, isothiazole, imidazole, indole, purine, quinoline, thiadiazole.

In a particular embodiment, at least one of X and Y is -C-R-, preferably both X and Y are -C-R-, wherein R is selected from hydrogen, an alkyl, an alkoxyl and a hydroxyl group. Preferably, R is selected from hydrogen and an alkoxyl group, even more preferably R is hydrogen.

In another particular embodiment, W is -(CH₂)ₙ-, wherein n is an integer selected from 5 and 6.

In another particular embodiment, A is -NH-.

In a particular embodiment, Z is a hydroxyl group.

In a preferred embodiment, the compounds of general formula (I) are selected from:
[1] *N*-Hydroxy-6-[(1,2,3,4-tetrahydroacridin-9-yl)amino]hexanamide, with the following structural formula: 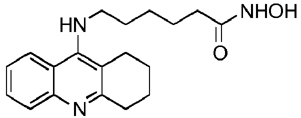
[2] *N*-Hydroxy-7-[(1,2,3,4-tetrahydroacridin-9-yl)amino]heptanamide, with the following structural formula: 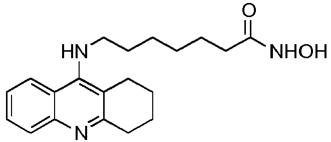
[3] 7-{(2,3-dihydro-1*H*-cyclopenta[*b*]quinolyl)amino}-*N*-hydroxyheptanamide, with the following structural formula: 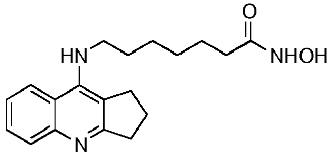
[4] *N-*Hydroxy-7-{(7,8,9,10-tetrahydro-6*H*-cyclohepta[*b*]quinolin-11-yl)amino} heptanamide, with the following structural formula: 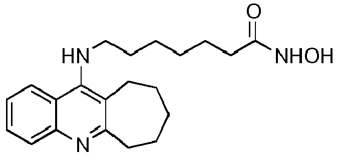
[5] *N*-Hydroxy-7-[(5-methoxy-1,2,3,4-tetrahydroacridin-9-yl)amino]heptanamide, with the following structural formula: 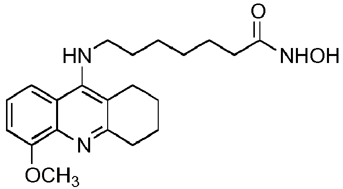
[6] *N*-Hydroxy-7-[(5-hydroxy-1,2,3,4-tetrahydroacridin-9-yl)amino]heptanamide, with the following structural formula: 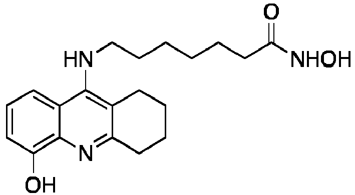
[7] *N*-Hydroxy-7-{(6,7,8,9-tetrahydrobenzo[*b*][1,8]naphthyridin-5-yl)amino} heptanamide, with the following structural formula: 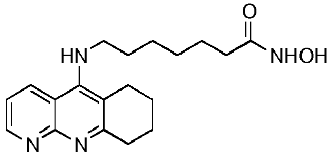
[8] *N*-Hydroxy-7-{(6,7,8,9-tetrahydrobenzo[*b*][1,7]naphthyridin-5-yl)amino} heptanamide, with the following structural formula: 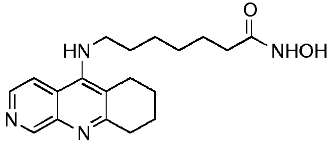
[9] *N*-Hydroxy-4-{[(1,2,3,4-tetrahydroacridin-9-yl)amino]methyl}benzamide, with the following structural formula: 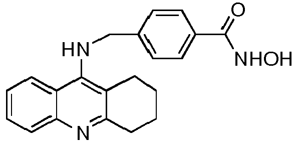
[10] *N*-[6-(hydroxyamino)-6-oxohexyl]-1,2,3,4-tetrahydroacridine-9-carboxamide, with the following structural formula: 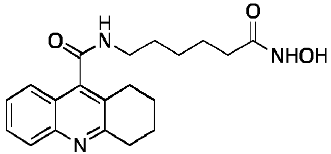
[11] *N*-[7-(hydroxyamino)-7-oxoheptyl]-1,2,3,4-tetrahydroacridine-9-carboxamide, with the following structural formula: 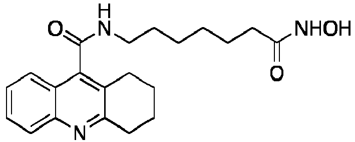
[12] *N*-(2-Amino-4-methylphenyl)-7-[(1,2,3,4-tetrahydroacridin-9-yl)amino] heptanamide, with the following structural formula: 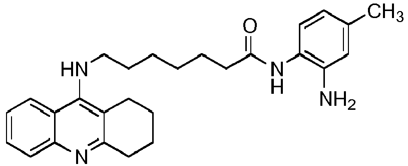
[13] *N*-(2-Amino-5-methylphenyl)-7-[(1,2,3,4-tetrahydroacridin-9-yl)amino] heptanamide, with the following structural formula: 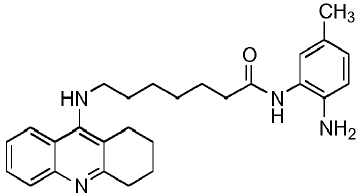
[14] *N*-[2-Amino-5-(*tert*-butyl)phenyl]-7-[(1,2,3,4-tetrahydroacridin-9-yl)amino] heptanamide, with the following structural formula: 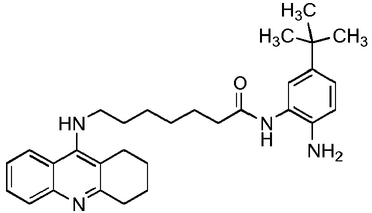
[15] *N*-(4-Amino-[1,1'-biphenyl]-3-yl)-7-[(1,2,3,4-tetrahydroacridin-9-yl)amino] heptanamide, with the following structural formula: 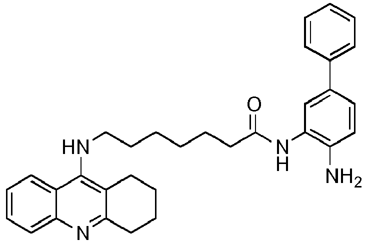
[16] *N*-(4-Amino-[1,1'-biphenyl]-3-yl)-6-[(1,2,3,4-tetrahydroacridin-9-yl)amino] hexanamide, with the following structural formula: 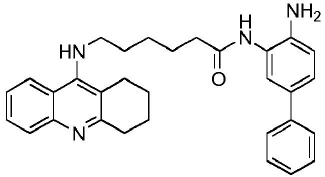
[17] *N-*(4-Amino-3'-methyl-[1,1'-biphenyl]-3-yl)-7-[(1,2,3,4-tetrahydroacridin-9-yl)amino]heptanamide, with the following structural formula: 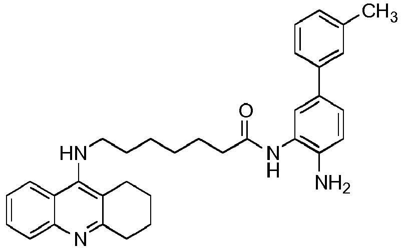
[18] *N*-{4-Amino-4'-(*tert-*butyl-[1,1'-biphenyl]-3-yl}-7-[(1,2,3,4-tetrahydroacridin-9-yl)amino]heptanamide, with the following structural formula: 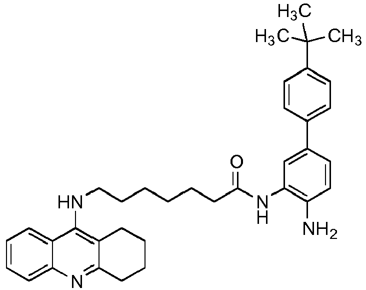
[19] *N*-[2-Amino-5-(naphthalen-2-yl)phenyl]-7-[(1,2,3,4-tetrahydroacridin-9-yl)amino] heptanamide, with the following structural formula: 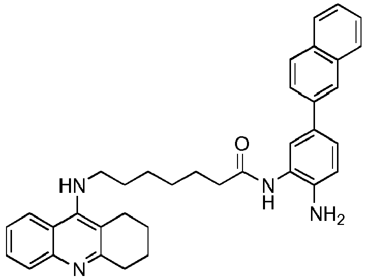
[20] *N-*(2-Amino-5-phenylpyridin-3-yl)-7-[(1,2,3,4-tetrahydroacridin-9-yl)amino] heptanamide, with the following structural formula: 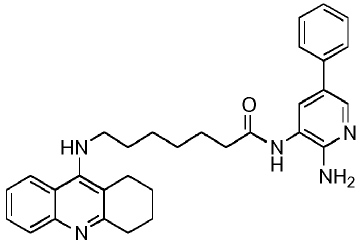
[21] *N*-[2-Amino-5-(pyridin-3-yl)phenyl]-7-[(1,2,3,4-tetrahydroacridin-9-yl)amino] heptanamide, with the following structural formula: 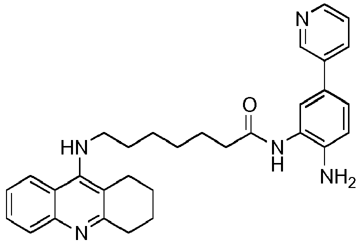
or a solvate or a salt or prodrug thereof.

The compounds of formula (I) defined above may be in the form of solvates or salts or prodrugs, preferably as a pharmaceutically acceptable species.

The term "pharmaceutically acceptable species" refers to compositions and molecular entities that are physiologically tolerable and do not typically produce an allergic reaction or a similar unfavorable reaction as gastric disorders, dizziness and suchlike, when administered to a human or animal. Preferably, the term "pharmaceutically acceptable" means it is approved by a regulatory agency of a state or federal government or is included in the U.S. Pharmacopoeia or other generally recognized pharmacopoeia for use in animals, and more particularly in humans.

The term "prodrug" is used in its broadest sense and encompasses those derivatives that are converted *in vivo* into the compounds of the invention. Experts in the art would readily produce such derivatives, and include, depending on the functional groups present in the molecule and without limitation, the following derivatives of the present compounds: disulfides, thioesters, esters, amino acid esters, phosphate esters, esters of metallic salt sulfonates, carbamates and amides.

The term "solvate" means any form of the active compound of the invention which has another molecule (for example a polar solvent such as water or ethanol, a cyclodextrin or a dendrimer) attached to it through noncovalent bonds. Methods of solvation are known within the art.

The invention also provides salts of the compounds of the invention. Nonlimiting examples are sulphates; hydrohalide salts; phosphates; lower alkane sulphonates; arylsulphonates; salts of C₁-C₂₀ aliphatic mono-, di- or tribasic acids which may contain one or more double bonds, an aryl nucleus or other functional groups such as hydroxy, amino, or keto; salts of aromatic acids in which the aromatic nuclei may or may not be substituted with groups such as hydroxyl, lower alkoxyl, amino, mono- or di- lower alkylamino sulphonamido. Also included within the scope of the invention are quaternary salts of the tertiary nitrogen atom with lower alkyl halides or sulphates, and oxygenated derivatives of the tertiary nitrogen atom, such as the N-oxides. In preparing dosage formulations, those skilled in the art will select the pharmaceutically acceptable salts.

Solvates, salts and prodrugs can be prepared by methods known in the state of the art. Note that the non-pharmaceutically acceptable solvates and prodrugs also fall within the scope of the invention because they can be useful in preparing pharmaceutically acceptable salts, solvates or prodrugs.

The compounds of the invention also seek to include compounds that differ only in the presence of one or more isotopically enriched atoms. For example, compounds having the present structures except for the replacement of a hydrogen by a deuterium or tritium, or the replacement of a carbon by a carbon enriched in ¹¹C, ¹³C or ¹⁴C or a ¹⁵N enriched nitrogen are within the scope of this invention.

### Synthesis of compounds of formula (I)

Another aspect of the invention refers to procedures to obtain compounds of general formula (I). The following methods A, B, C and D describe suitable procedures to obtain compounds of formula (Ia), (Ib), (Ic) and I(d), respectively, or solvates or salts or prodrugs thereof.

Compounds of formula (Ia) correspond to compounds of formula (I), wherein X, Y, W and n have the meaning given above, A is NH and Z is OH.

Compounds of formula (Ib) correspond to compounds of formula (I), wherein X, Y, W and n have the meaning given above, A is NH and Z is a 2'-aminoaryl or 2'-aminoheteroaryl group in the form described by structure (III): 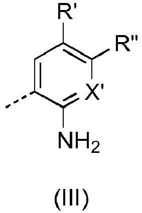 wherein X', R' and R" have the meaning given above.

Compounds of formula (Ic) correspond to compounds of formula (I), wherein X, Y, W and n have the meaning given above, A is C(O)NH and Z is OH.

Compounds of formula (Id) correspond to compounds of formula (I), wherein X, Y, W and n have the meaning given above, A is C(O)NH and Z is a 2'-aminoaryl or 2'-aminoheteroaryl group in the form described by structure (III) above, wherein X', R' and R" have the meaning given above.

### Method A

Method A describes the procedure for obtaining compounds of general formula (Ia), 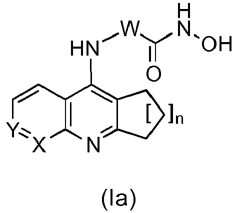 wherein X, Y, n and W have the meaning given above,
which comprises:
a) reacting an amino acid of general formula (IV), 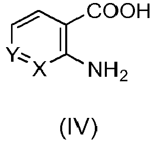 wherein X and Y have the meaning given above;
   with a cyclic ketone of general formula (V), 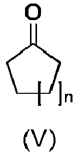 wherein n has the meaning given above;
   in the presence of an appropriate chlorination-condensation reagent, to afford a compound of formula (VI): 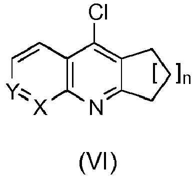 wherein X, Y and n have the meaning given above;
b) reacting the compound of formula (VI) with an ester of general formula X⁻H₃N⁺-W-COOR"', wherein W has the meaning indicated above, R"' is a linear or branched alkyl group, and X⁻ is an organic or inorganic anion,
   in the presence of an organic base, and an appropriate solvent,
   to afford a compound of formula (VII): 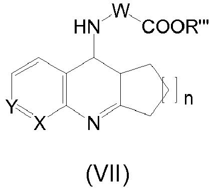 wherein X, Y, n, W and R"' have the meaning given above;
c) reacting the compound of formula (VII) with hydroxylamine hydrochloride, in the presence of a liquid alcohol, a solution of a metallic alkoxide in the previously indicated alcohol, and an acid-base indicator.

For the aims of the invention, the reaction mixture of step a) can be made by adding one of the compounds of formula (IV) and (V) to the other and cooling to 0-5 °C. The chlorination-condensation reagent can be added dropwise at 0-5 °C and the resulting mixture can be heated at a temperature comprised between +100 °C and +185 °C until completion of the reaction.

As chlorination-condensation reagent, the use of phosphorous oxychloride (phosphoryl chloride) is preferred.

Compound (VI) can be isolated by evaporation at reduced pressure and addition of an organic solvent such as ethyl acetate, followed by basification by means of an adequate inorganic base. This inorganic base may be selected from the group consisting of carbonates of alkaline metals or alkaline earth metals (e.g. sodium, lithium, potassium, calcium, or magnesium carbonate), bicarbonates of alkaline metals (e.g. sodium, lithium or potassium bicarbonate), sulfates of alkaline metals or alkaline earth metals (e.g. sodium, lithium, potassium, calcium, or magnesium sulfate), acetates of alkaline metals or alkaline earth metals (e.g. sodium, lithium, potassium, calcium, or magnesium acetate), hydroxides of alkaline metals or alkaline earth metals (e.g. sodium, lithium, potassium, calcium, or magnesium hydroxide) or phosphates, monohydrogen phosphates or dihydrogen phosphates of alkaline metals or alkaline earth metals (e.g. sodium, lithium, potassium, calcium, or magnesium phosphate, or potassium dihydrogen phosphate).

Separation and dehydration of the organic phase, followed by evaporation of the organic solvent, yields compound (VI) which can be used as such in next step b).

In step b) of the Method A, the compound of formula (VI) is reacted with an ester of formula X-H₃N+-W-COOR"', wherein W has the meaning indicated above, R'" is a linear or branched alkyl group, and X- is an organic or inorganic anion, in the presence of an organic base and an appropriate solvent.

In a preferred embodiment, R'" is selected from methyl, ethyl and tert-butyl.

Examples of organic or inorganic anion X- include halide, sulfate, perchlorate, acetate, tartrate or other carboxylic acid.

Step b) can be made by adding the organic base and the solvent on a mixture of compound of formula (VI) and the ester at room temperature. After completion of the addition, the resulting mixture can be stirred and heated either by external thermal heating or by microwave irradiation at a temperature ranging from +60 °C to +145 °C until completion of the reaction.

The organic base may be a primary, secondary or tertiary amine, preferably a tertiary amine selected from among the cyclic or acyclic aliphatic amines with C₃-C₁₀ carbon atoms and the alkanoaromatic amines with C₉-C₁₅ carbon atoms, more preferably *N,N*-dimethylaniline, triethylamine, *N,N-*diisopropyl ethylamine (DIPEA), *N-*methyl morpholine, *N*-methylpyrrolidine, 1,8-diazabicyclo[5.4.0] undec-7-ene (DBU) and pyridine.

The solvent can be a nonpolar solvent such as a linear or branched aliphatic hydrocarbon of C₆-C₁₀ carbons or an aromatic hydrocarbon such as toluene, xylene (any mixture of isomers) or similar.

After standard work-up, the resulting ester of formula (VII) can be isolated and transformed into the corresponding hydroxamic acid of formula (Ia) according to step c).

Said step c) can be performed by adding one of the hydroxylamine hydrochloride, the liquid alcohol and the acid-base indicator to a mixture formed by the other two components at a temperature ranging from 0 °C to +30 °C. After completion of the addition, an aliquot of the alcoholic solution of the metallic alkoxide taken from a stock solution is added drowpwise until the acid-base indicator changes its color thus showing a basic pH in the resulting reaction mixture. To this latter mixture the ester (VII) and the alcoholic solution of the metal alcoxide in excess are added and the resulting mixture is stirred at a temperature ranging from 0 °C to +30 °C until the completion of the reaction.

The alcohol can be selected among any alkyl alcohol liquid at room temperature and the acid-base indicator can be any compound whose change in color permits to detect unambiguously a basic pH under the indicated reaction conditions. Suitable examples are phenolphthalein, thymolphthalein, thymol blue, nile blue, diazo violet, bromocresol purple, dimethyl yellow, and similar compounds.

The synthetic route mentioned above is outlined in the following scheme:

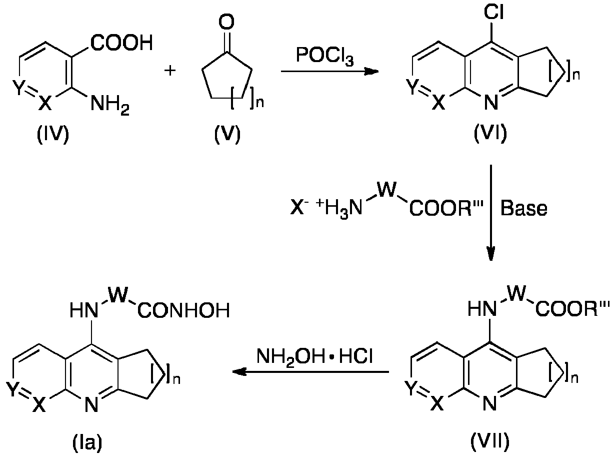

### Method B

Method B represents a procedure for the preparation of compounds of general formula (Ib): 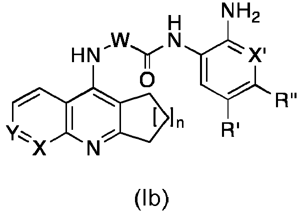 wherein X, Y, n and W have the meaning given in the description of Method A and above, and X', R' and R" have the meaning given above,
which comprises:
a) reacting an ester of general formula (VII) prepared according to Method A, 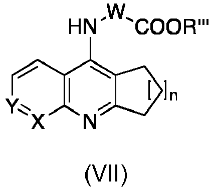 wherein X, Y, n and W have the meaning given above, and R"' has the meaning given in Method A,
   with an inorganic hydroxide dissolved or suspended in the appropriate volume of water, in the presence of a polar protic solvent,
   to afford a compound of formula (VIII): 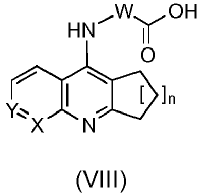 wherein X, Y, n and W have the meaning given above;
b) reacting the compound of formula (VIII) with a protected diamine of general formula (IX), 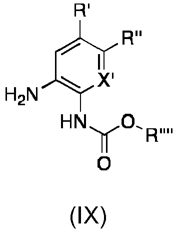 wherein
   X', R' and R" have the meaning given above;
   R"" is a tert-butyl, a benzyl or a 9-fluorenemethyl group,
   in the presence of an organic base, a coupling reagent, and an appropriate solvent,
   to afford the compound of formula (X): 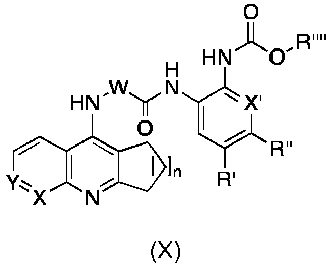 wherein the meaning of X, Y, n, W, X', R', R" and R"" are given above,
c) deprotecting the compound of formula (X) in the presence of an appropriate deprotecting agent and an appropriate solvent, to afford the compound of formula (Ib).

For the aims of the invention, the reaction of step a) can be made by mixing in any order the different reactants at a temperature ranging from 0 °C to +25 °C. The resulting mixture is stirred at a temperature ranging from +80 °C to +120 °C until the completion of the reaction.

The polar protic solvent can be selected among any liquid alcohol at room temperature. The inorganic hydroxide can be selected among the usual alkaline metals such as lithium, sodium or potassium.

The obtained carboxylic acid of formula (VIII) is subjected to a coupling reaction in step b). Said step can be carried out by mixing in any order the different compounds (compound of formula (VIII), compound of formula (IX), organic base, coupling reagent and solvent) under inert atmosphere and at a temperature ranging from 0 °C to +30 °C. The resulting mixture is stirred for 8-24 hours at a temperature comprised in the range from +10 °C to +30 °C.

The organic base can be selected among a primary, secondary or tertiary amine, preferably a tertiary amine selected from among the cyclic or acyclic aliphatic amines with C₃-C₁₀ carbon atoms and the alkanoaromatic amines with C₉-C₁₅ carbon atoms, more preferably N,N-dimethylaniline, triethylamine, N,N-diisopropyl ethylamine (DIPEA), N-methyl morpholine, N-methylpyrrolidine, 1,8-diazabicyclo[5.4.0] undec-7-ene (DBU) and pyridine.

The coupling reagent can be selected among amide coupling reagents such as 1-ethyl-3-(3-dimethylaminopropyl)carbodiimide (EDCI), *O*-(7-azabenzotriazol-1-yl)-*N,N,N',N*'-tetramethyluronium hexafluorophosphate (HATU), *O*-(benzotriazole)-*N,N,N',N'*-tetramethyluronium hexafluorophosphate (HBTU), *O*-(benzotriazol-1-yl)-*N,N,N',N'*-tetramethyluronium tetrafluoroborate (TBTU), propylphosphonic anhydride (T3P), (benzotriazol-1-yloxy)tris(dimethylamino)phosphonium hexafluorophosphate (BOP).

The solvent can be a polar nonprotic solvent such a cyclic or acyclic ether, *N,N-*dimethylformamide or 1,2-dimethoxyethane.

The product of the above indicated reaction is a protected benzamide of general formula (X).

The final step leading to the preparation of compounds of general formula (Ib), step c), comprises the deprotection of the compound of formula (X) by reacting said compound with an appropriate deprotecting reagent in the presence of an appropriate solvent.

This step c) can be made by mixing the compound of formula (X) and the solvent at a temperature ranging from 0 °C to +30 °C. To this mixture, the deprotecting reagent can be added at a temperature ranging from 0 °C to +30 °C and the resulting mixture is stirred until completion of the deprotection reaction.

The solvent can be a polar protic solvent such an alcohol, like ethanol or other liquid alcohol at room temperature, a polar nonprotic solvent such a cyclic or acyclic ether, N,N-dimethylformamide, dichloromethane, 1,2-dichloroethane, 1,2-dimethoxyethane or similar.

The suitable deprotecting reagent can vary depending on the nature of the carbamate moiety present in the compound of formula (X). If R"" is a tert-butyl group (C(=O)OR"" being a *Boc* group) the deprotecting reagent can be a suitable acid such as trifluoroacetic acid or similar; if R"" is a benzyl group (C(=O)OR"" being a *Cbz* group) the deprotecting reagent is a hydrogenation system formed by hydrogen gas in the presence of a suitable heterogeneous or homogeneous catalyst such as Pd/C or similar; if R"" is a 9-fluorenemethyl group (C(=O)OR"" being a Fmoc group) the deprotecting reagent is a suitable base such as piperidine or similar.

The synthetic route mentioned above is outlined in the following scheme:

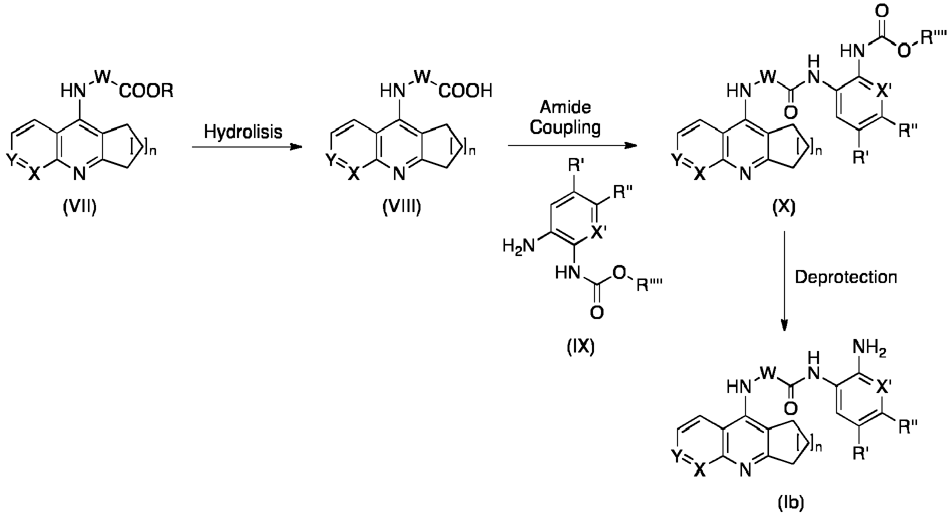

### Method C

Method C represents a procedure for the preparation of compounds of general formula (Ic): 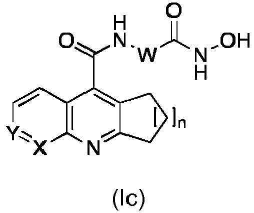 wherein X, Y, n and W have the meaning given above,
which comprises:
a) reacting a compound of formula (XI), 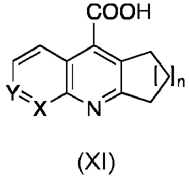 wherein X, Y and n have the meaning given above;
   with an ester of general formula X⁻H₃N⁺-W-COOR"', wherein W, R"' and X⁻ have the meaning indicated in Method A, in the presence of an organic base, a coupling reagent, and an appropriate solvent,
   to afford a compound of formula (XII): 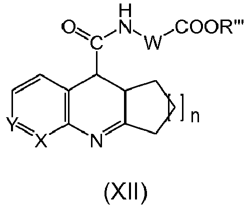 wherein X, Y, n, W and R"' have the meaning given above; and
b) reacting the compound of formula (XII) with hydroxylamine hydrochloride, in the presence of a liquid alcohol and a solution of a metallic alkoxide in the previously indicated alcohol and an acid-base indicator.

For the aims of the invention, the reaction of step a) can be made by mixing in any order the different reactants (compound of formula (XI), the ester, the organic base, the coupling agent and the solvent) at a temperature ranging from -85 °C to +30 °C. The resulting mixture is stirred for 8-24 hours at a temperature comprised in the range from +0 °C to +30 °C.

The organic base can be selected among a primary, secondary or tertiary amine, preferably a tertiary amine selected from among the cyclic or acyclic aliphatic amines with C₃-C₁₀ carbon atoms and the alkanoaromatic amines with C₉-C₁₅ carbon atoms, more preferably *N,N*-dimethylaniline, triethylamine, *N,N*-diisopropyl ethylamine (DIPEA), N-methyl morpholine, N-methylpyrrolidine, 1,8-diazabicyclo[5.4.0] undec-7-ene (DBU) and pyridine.

The coupling reagent can be selected among amide coupling reagents such as oxalyl chloride, phenyl dichlorophosphate, diethyl cyanophosphonate (DEPC), or the 1-hydroxybenzotriazole (HOBt) and *N*-(3-dimethylaminopropyl)-*N*'-ethylcarbodiimide (EDC) system, 1-ethyl-3-(3-dimethylaminopropyl)carbodiimide (EDCI), *O-*(7-azabenzotriazol-1-yl)-*N,N,N',N*'-tetramethyluronium hexafluorophosphate (HATU), *O-*(benzotriazole)-*N,N,N',N'-*tetramethyluronium hexafluorophosphate (HBTU), *O-*(benzotriazol-1-yl)-*N,N,N',N'*-tetramethyluronium tetrafluoroborate (TBTU), propylphosphonic anhydride (T3P), (benzotriazol-1-yloxy)tris(dimethylamino)phosphonium hexafluorophosphate (BOP).

The solvent can be a polar nonprotic solvent such a cyclic or acyclic ether, *N,N-*dimethylformamide or 1,2-dimethoxyethane.

After standard work-up, the ester (XII) can be isolated and transformed into the corresponding hydroxamic acid of formula (Ic) according to step b).

Said step b) can be performed by adding one of the hydroxylamine hydrochloride, the liquid alcohol and the acid-base indicator to a mixture formed by the other two components at a temperature ranging from 0 °C to +30 °C. After completion of the addition, an aliquot of the alcoholic solution of the metallic alkoxide taken from a stock solution is added drowpwise until the acid-base indicator changes its color thus showing a basic pH in the resulting reaction mixture. To this latter mixture the ester (XII) and the alcoholic solution of the metal alcoxide in excess are added and the resulting mixture is stirred at a temperature ranging from 0 °C to +30 °C until the completion of the reaction.

The alcohol can be selected among any alkyl alcohol liquid at room temperature and the acid-base indicator can be any compound whose change in color permits to detect unambiguously a basic pH under the indicated reaction conditions. Suitable examples are phenolphthalein, thymolphthalein, thymol blue, nile blue, diazo violet, bromocresol purple, dimethyl yellow, and similar compounds.

The synthetic route mentioned above is outlined in the following scheme:

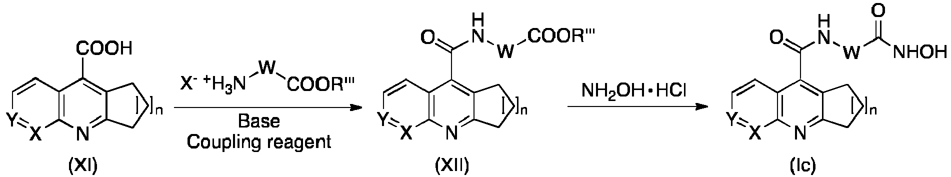

### Method D

Method D represents a procedure for the preparation of compounds of general formula (Id): 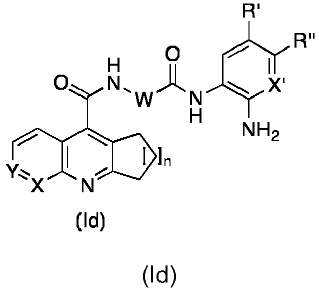 wherein X, Y, n and W have the meaning given in the description of Method A and above, and X', R' and R" have the meaning given in the description of Method B, which comprises:
a) reacting an ester of general formula (XII) prepared according to Method C, with an inorganic hydroxide dissolved or suspended in the appropriate volume of water, in the presence of a polar protic solvent, to afford the compound of formula (XIII): 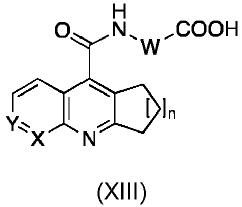 wherein X, Y, n and W have the meaning given above;
b) reacting a compound of formula (XIII) with a protected diamine of general formula (IX): 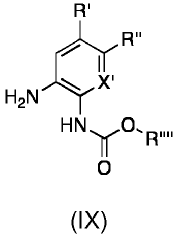 wherein
   X', R' and R" have the meaning given above; and
   R"" is a tert-butyl, a benzyl or a 9-fluorenemethyl group,
   in the presence of an organic base, a coupling reagent, and an appropriate solvent, to afford the compound of formula (XIV): 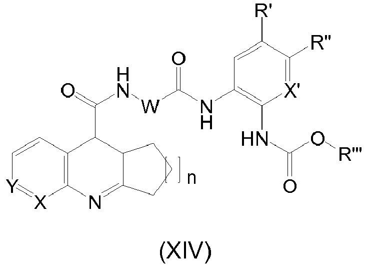 wherein X, Y, n, W, X', R', R" and R"" have the meaning given above;
c) deprotecting the compound of formula (XIV) in the presence of an appropriate deprotecting agent and an appropriate solvent, to afford the compound of formula (Id).

For the aims of the invention, the reaction of step a) can be made by mixing in any order the different reactants (compound of formula (XII), the inorganic hydroxide dissolved or suspended in water and the polar solvent) at a temperature ranging from 0 °C to +25 °C. The resulting mixture is stirred at a temperature ranging from +80 °C to +120 °C until the completion of the reaction.

The polar protic solvent can be selected among any liquid alcohol at room temperature. The inorganic hydroxide can be selected among the usual alkaline metals such as lithium, sodium or potassium.

The obtained carboxylic acid of formula (XIII) is subjected to a coupling reaction in step b). Said step can be carried out by mixing in any order the different components (the compound of formula (XIII), the compound of formula (IX), the organic base, the coupling agent and the solvent) under inert atmosphere and at a temperature ranging from 0 °C to +30 °C. The resulting mixture is stirred for 8-24 hours at a temperature comprised in the range from +10 °C to +30 °C.

The organic base can be selected among a primary, secondary or tertiary amine, preferably a tertiary amine selected from among the cyclic or acyclic aliphatic amines with C₃-C₁₀ carbon atoms and the alkanoaromatic amines with C₉-C₁₅ carbon atoms, more preferably *N,N*-dimethylaniline, triethylamine, *N,N-*diisopropyl ethylamine (DIPEA), *N*-methyl morpholine, *N*-methylpyrrolidine, 1,8-diazabicyclo[5.4.0] undec-7-ene (DBU) and pyridine.

The coupling reagent can be selected among amide coupling reagents such as 1-ethyl-3-(3-dimethylaminopropyl)carbodiimide (EDCI), *O*-(7-azabenzotriazol-1-yl)-*N,N,N',N'*-tetramethyluronium hexafluorophosphate (HATU), O-(benzotriazole)-*N,N,N',N*'-tetramethyluronium hexafluorophosphate (HBTU), *O*-(benzotriazol-1-yl)-*N,N,N',N*'-tetramethyluronium tetrafluoroborate (TBTU), propylphosphonic anhydride (T3P), (benzotriazol-1-yloxy)tris(dimethylamino)phosphonium hexafluorophosphate (BOP).

The solvent can be a polar nonprotic solvent such a cyclic or acyclic ether, *N,N-*dimethylformamide or 1,2-dimethoxyethane.

The product of the above indicated reaction is a protected benzamide of general formula (XIV).

The final step leading to the preparation of compounds of general formula (Id), step c), comprises the deprotection of the compound of formula (XIV) by reacting said compound with an appropriate deprotecting reagent in the presence of an appropriate solvent.

This step c) can be made by mixing the compound of formula (XIV) and the solvent at a temperature ranging from 0 °C to +30 °C. To this mixture, the deprotecting reagent can be added at a temperature ranging from 0 °C to +30 °C and the resulting mixture is stirred until completion of the deprotection reaction.

The solvent can be a polar protic solvent such an alcohol, like ethanol or other liquid alcohol at room temperature, a polar nonprotic solvent such a cyclic or acyclic ether, *N,N*-dimethylformamide, dichloromethane, 1,2-dichloroethane, 1,2-dimethoxyethane or similar.

The suitable deprotecting reagent can vary depending on the nature of the carbamate moiety present in the compound of formula (XIV). If R"" is a *tert*-butyl group (C(=O)OR"" being a *Boc* group) the deprotecting reagent can be a suitable acid such as trifluoroacetic acid or similar; if R"" is a benzyl group (C(=O)OR"" being a *Cbz* group) the deprotecting reagent is a hydrogenation system formed by hydrogen gas in the presence of a suitable heterogeneous or homogeneous catalyst such as Pd/C or similar; if R"" is a 9-fluorenemethyl group (C(=O)OR"" being a *Fmoc* group) the deprotecting reagent is a suitable base such as piperidine or similar.

The synthetic route mentioned above is outlined in the following scheme:

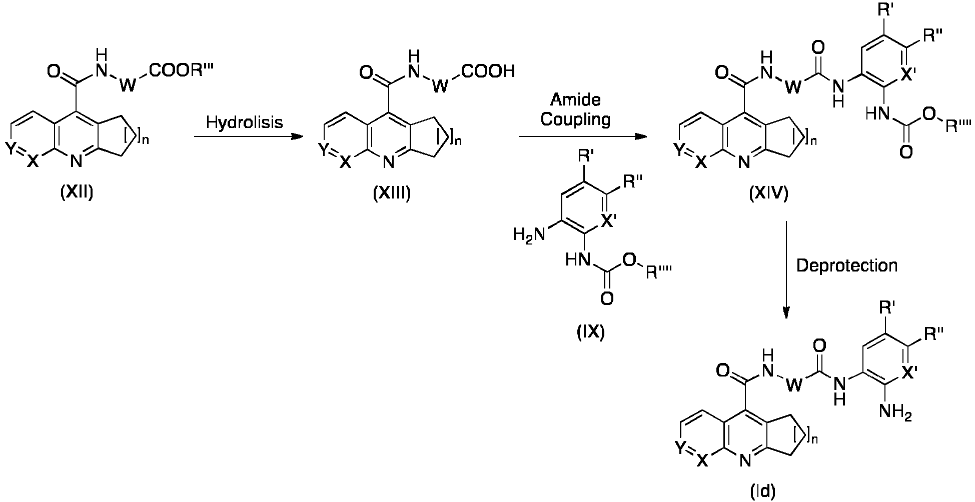

The initial compounds and starting materials, e.g. the compounds of formula (IV), (V) and (IX), are either commercially available or can be obtained following procedures described in the literature. For example, see O. Moradel et al. PCT/US2007/066045 (WO/2007/118137) and L.H. Tsai et al. PCT/US2009/006355 (WO/2010/065117).

A further embodiment of the invention is a salt or solvate or prodrug thereof of a compound of formula (I). According to a particular embodiment, the salt is a phenoxy salt of alkaline metals or alkaline earth metals. To obtain the salts corresponding to compounds of formula (Ia) and (Ic), the hydroxyl group can be treated with hydroxides of alkaline metals or alkaline earth metals (e.g. sodium, lithium, potassium, calcium, or magnesium hydroxide) at a temperature ranging from 0°C to +40°C. In an embodiment of the invention the reaction takes place at room temperature using water as solvent. To obtain the salts corresponding to compounds of formula (Ib) and (Id), the amino group can be treated with organic or inorganic acids. In an embodiment of the invention these acids can be selected among the usual acids of acceptable pharmacological use. To obtain these salts the amino group can be treated directly with the corresponding acid in an appropriate solvent at a temperature ranging from 0 °C to +30 °C. In the product thus formed, the anion associated with the corresponding salt can be chloride, acetate, tartrate, lactate, or similar.

### Use of the compounds of the invention

According to a particular embodiment, the compounds of general formula (I) are useful for the treatment of various types of cancer, hematological malignancy, proliferative diseases, neurological disorders and immunological disorders, by changing the acetylation pattern of histones involved in the mentioned diseases.

According to a particular embodiment, the cancer is selected from breast cancer, chronic myelogenous (or myeloid) leukemia (CML), colorectal cancer, fibrosarcoma, gastric cancer, glioblastoma, kidney cancer, liver cancer, lung cancer, melanoma, nasopharyngeal cancer, oral cancer, orthotopic multiple myeloma, osteosarcoma, ovarian cancer, pancreatic cancer, and prostate cancer.

According to an embodiment of the invention the neurological disorder is schizophrenia, fragile X syndrome or Alzheimer.

According to an embodiment of the invention the immunological disorder is proviral latency of human immunodeficiency virus type 1 (HIV-1).

### Pharmaceutical compositions

Another aspect of the present invention refers to a pharmaceutical composition which comprises the compounds of formula (I) of the invention, or a pharmaceutically acceptable solvate or salt or prodrug thereof, and at least a pharmaceutically acceptable excipient.

The term "excipient" refers to a vehicle, diluent or adjuvant that is administered with the active ingredient. Such pharmaceutical excipients can be sterile liquids, such as water and oils, including those of petroleum, animal, vegetable or synthetic origin, such as peanut oil, soybean oil, mineral oil, sesame oil and similars. Water or saline aqueous solutions and aqueous dextrose and glycerol solutions, particularly for injectable solutions, are preferably used as vehicles. Suitable pharmaceutical vehicles are described in "Remington's Pharmaceutical Sciences" by E.W. Martin, 21st Edition, 2005; or "Handbook of Pharmaceutical Excipients", Rowe C. R.; Paul J. S.; Marian E. Q., sixth Edition.

Examples of pharmaceutical compositions include any solid composition (tablets, pills, capsules, granules, etc.) or liquid composition (solutions, suspensions or emulsions) for oral, topical or parenteral administration.

In a preferred embodiment the pharmaceutical compositions are in oral delivery form. Pharmaceutical forms suitable for oral administration may be tablets and capsules and may contain conventional excipients known in the art such as binders, for example syrup, gum arabic, gelatin, sorbitol, tragacanth or polyvinylpyrrolidone; fillers, for example lactose, sugar, cornstarch, calcium phosphate, sorbitol or glycine; lubricants for the preparation of tablets, for example magnesium stearate; disintegrants, for example starch, polyvinylpyrrolidone, sodium starch glycolate or microcrystalline cellulose; or pharmaceutically acceptable wetting agents such as sodium lauryl sulphate.

Solid oral compositions can be prepared by conventional methods of blending, filling or preparation of tablets. Repeated blending operations can be used to distribute the active ingredient in all the compositions that use large amounts of fillers. Such operations are conventional in the art. The tablets can be prepared, for example, by dry or wet granulation and optionally can be coated by well known methods in normal pharmaceutical practice, in particular using a enteric coating.

Pharmaceutical compositions can also be adapted for parenteral administration, such as sterile solutions, suspensions or lyophilized products in the appropriate unit dosage form. Suitable excipients such as fillers, buffering agents or surfactants can be used.

The mentioned formulations will be prepared using standard methods such as those described or referred to in the Spanish and U.S. Pharmacopoeias and similar reference texts.

In general, the effective amount of a compound of the invention to be administered will depend on the relative efficacy of the compound chosen, the severity of the disorder being treated and the patient's weight. However, the active compounds will normally be administered one or more times a day, for example 1, 2, 3 or 4 times daily, with typical total daily doses in the range from 0.01 up to 1000 mg/kg/day.

The compounds of the present invention can be used with at least another drug to provide a combination therapy. This other drug or drugs may be part of the same composition, or may be provided as a separate composition and can be administered at the same time or at different times.

The term "treatment" or "treating" in the context of this document means administration of a compound or a formulation according to this invention to prevent, improve or eliminate the disease or one or more symptoms associated with the disease. "Treatment" also encompasses preventing, improving or eliminating the physiological sequelae of the disease.

In order to facilitate the understanding of the preceding ideas, some examples of experimental procedures and embodiments of the present invention are described below. These examples are merely illustrative.

### EXAMPLES

### General Synthesis Methods

### Method A

### a.1) Synthesis of tricyclic caps

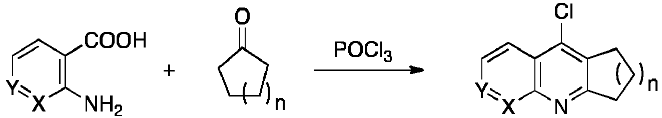

A mixture of the corresponding aminoacid (46.53 mmol) and the corresponding cyclic ketone (51.70 mmol) under argon atmosphere was cooled to 0°C. Then 38.75 ml of POCl₃ were added dropwise, and the resulting mixture was refluxed for 16h. Then the crude reaction solution was evaporated to dryness under reduced pressure. The resulting mixture was poured onto a mixture of ice and water, and ethyl acetate was added. Then solid Na₂CO₃ was added slowly until basic pH was reached. The organic layer was decanted and the aqueous phase was extracted with AcOEt. The combined organic phases were dried over Na₂SO₄ and evaporated under reduced pressure, to yield the corresponding product with high purity (60-92% yield).

### a.2) Synthesis of linear spacers

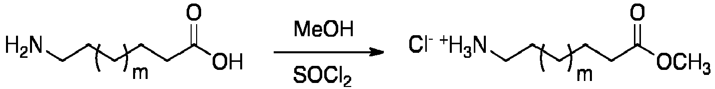

In a round-bottom flask under argon atmosphere methanol (34.4 ml) was introduced and cooled down to 0°C, and SOCl₂ (7.26 ml) was added dropwise. The corresponding aminoacid (34.43 mmol) was added, and the mixture was stirred at room temperature for 16h. Then the crude reaction solution was evaporated to dryness under reduced pressure.

### a.3) Coupling of caps and spacers under classical heating

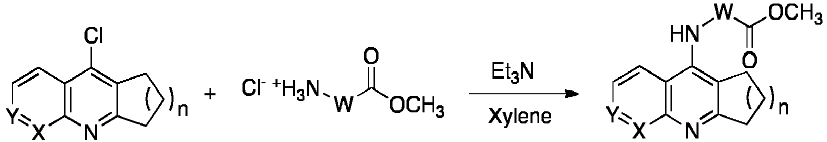

To a mixture of the corresponding cap (1.0 mmol) and the corresponding spacer (2.0 mmol) under argon atmosphere, xylene (1 ml) and Et₃N (0.4 ml) were added, and the mixture was refluxed for 6 hours. Then, xylene was evaporated under reduced pressure, and the crude thus obtained was purified by flash chromatography (silica gel, EtOAc/Hx and MeOH/CH₂Cl₂) to afford the desired product (70-76% yield).

### a.4) Coupling of caps and spacers under microwave irradiation

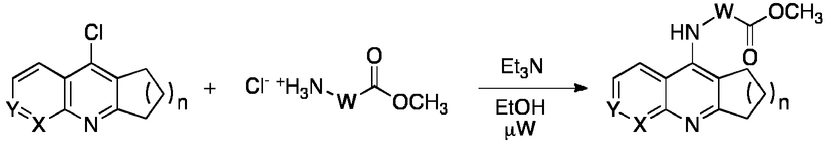

A mixture of the corresponding cap (1.0 mmol) and the corresponding spacer (2.0 mmol) was placed in a microwave vessel. Then, EtOH (1 ml) and Et₃N (0.4 ml) were added, and the mixture was irradiated with microwaves in a Biotage Initiator focused microwave reactor at 140 °C for 3 hours. Then, ethyl acetate was added, and the organic layer was washed with water, dried over sodium sulfate, filtered and evaporated under reduced pressure. The crude thus obtained was purified by flash chromatography (silica gel, EtOAc/Hx and MeOH/CH₂Cl₂) to afford the desired product (90-95% yield).

### a.5) Synthesis of the hydroxamic acids

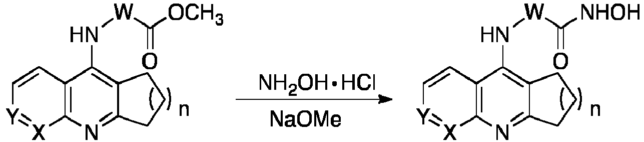

To a solution of hydroxylamine hydrochloride (3.75 mmol) and phenolphtalein (1 mg) in methanol (80 ml) under inert atmosphere, an aliquot of sodium methoxide in methanol (taken from a solution of 0.65 g, 12 mmol of sodium methoxide in 3.3 ml of methanol) was added dropwise until a permanent pink color was observed. The corresponding methyl ester (0.61 mmol) and sodium methoxide in methanol (5.0 mmol, 1.4 ml of the previously prepared solution) were subsequently added. The reaction mixture was stirred for 26h, the formation of a dense precipitate being observed. Water (3 ml) was added, and this solution was acidified with glacial acetic acid and extracted with CH₂Cl₂ (3 x 10 ml). The combined organic fractions were dried over Na₂SO₄ and evaporated under reduced pressure, to obtain the corresponding hydroxamic acid (80-95% yield).

### Method B

### b.1) Hydrolysis

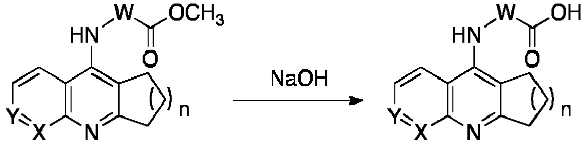

A mixture of the corresponding methyl ester (15.5 mmol), ethanol (176 ml) and 10% aqueous NaOH (85.8 ml) was refluxed for 45 minutes. Then, solvents were evaporated under reduced pressure, and the crude thus obtained was purified by flash chromatography (silica gel, EtOAc/Hx and MeOH/CH₂Cl₂) to afford the desired product (40-80% yield).

### b.2) Synthesis of benzamide chelating groups

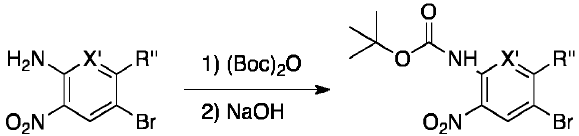

To a solution of corresponding aniline (46.1 mmol) and Boc-anhydride (92.2 mmol) in THF (100ml) stirred at room temperature was added a catalitic amount of 4-(dimethylamino)pyridine (DMAP). The reaction mixture was allowed to stir for 90 min, the solvent was removed *in vacuo* and the residue was dried under vacuum. The oil thus obtained was disolved in THF (46 ml), treated with an aqueous sodium hydroxide solution (2N, 46 ml) and heated to 65°C for 18h. Solid sodium hydroxide (1.8 g, 46.1 mmol) was added to the reaction mixture and heating was continued for 4h; then THF was removed *in vacuo* and a yellow solid precipitated from the aqueous solution. The solid was filtered, washed with H₂O and dried under vacuum to afford the N-Boc protected aniline (Yield 90-99%).

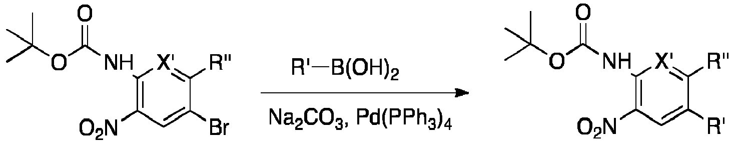

A mixture of the corresponding tert-butyl carbamate (0.62 mmol), the corresponding boronic acid (0.74 mmol), sodium carbonate (0.93 mmol) and Pd(PPh₃)₄ (0.04 mmol) in DME/H₂O (2:1, 5 ml) was vigorously stirred at 110°C under argon atmosphere for 20h. Then water was added, and the product was extracted with ethyl acetate. The combined organic layers were washed with water, dried over sodium sulfate, filtered and concentrated. The residue was purified by flash chromatography (silica gel, 10% EtOAc/Hx) to afford the desired product (89-95% yield).

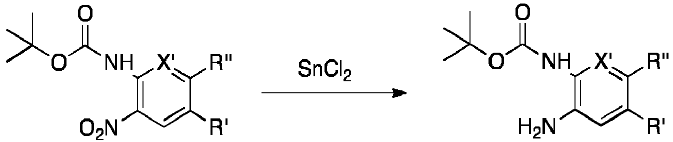

A mixture of the corresponding nitro compound (4.76 mmol), SnCl₂ (33.32 mmol) and DMF (34 ml) was placed in a round bottom flask under argon atmosphere. The mixture was stirred at 50 °C for 1.5 h. Then ethyl acetate was added, and the organic layer was washed with water and Na₂CO₃ 10% aqueous solution, dried over sodium sulfate, filtered and evaporated under reduced pressure, yielding the desired product (50% yield, brown solid).

### b.3) Coupling of the benzamide chelating group

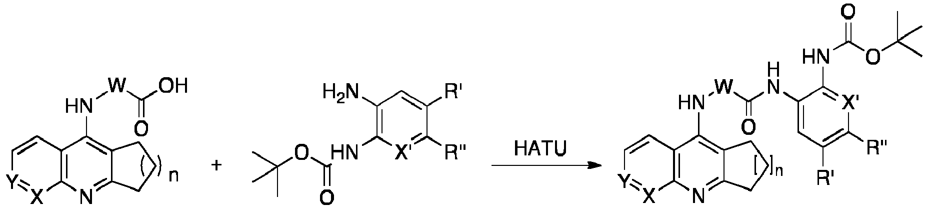

A mixture of the corresponding carboxylic acid (0.508 mmol), the corresponding tert-butyl aminecarbamate (0.508 mmol), DIPEA (0.7 ml, 4.08 mmol) and HATU (1.14 mmol) in DMF (5 ml) was stirred for 16 h under argon atmosphere. Then solvents were removed by evaporation. The residue was diluted with EtOAc and washed with water. The organic layer was dried over sodium sulfate, filtered and concentrated. The crude thus obtained was purified by flash chromatography (silica gel, 10% EtOAc/Hx) to give the desired product (45-50% yield).

### b.4) Deprotection of the carbamate moiety

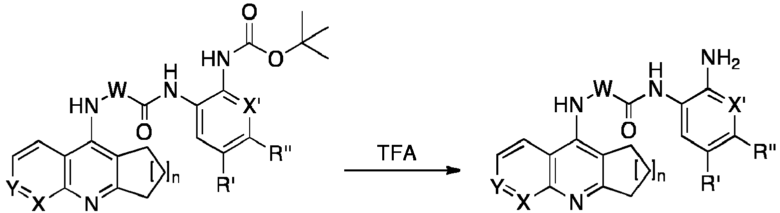

A mixture of the corresponding N-Boc protected compound (0.42 mmol), CH₂Cl₂ (3.22 ml) and TFA (1.05 ml) was stirred at room temperature for 16 hours. Then solvents were removed by evaporation. The residue was diluted with CH₂Cl₂, and washed with an ice/ NaHCO₃ mixture. The organic layer was dried over sodium sulfate, filtered and concentrated. The crude thus obtained was purified by flash chromatography (silica gel, EtOAc/Hx and MeOH/CH₂Cl₂) to obtain the desired product (50-80% yield).

### Method C

### c.1) Coupling of caps and spacers

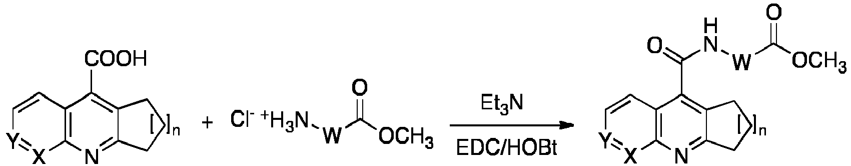

A solution of the corresponding carboxylic acid (2.2 mmol) and the corresponding spacer (2.2 mmol) in DMF was cooled to 0°C. Triethylamine (1.73 ml, 12.32 mol), 1-hydroxybenzotriazole (0.33 g, 2.42 mmol), *N*-(3-dimethylaminopropyl)-*N*'-ethylcarbodiimide hydrochloride (0.46 g, 2.42 mmol) and N-methylmorpholine (0.24 ml, 2.2 mmol) were added subsequently, and the mixture was stirred for 2h at 0°C, and for an additional 96h at room temperature. Ethyl acetate (160 ml) was added, and the obtained solution was washed with water (30 ml), Na₂S₂O₃ 1N (30 ml, aqueous solution), water (30 ml), NaHCO₃ (30 ml, saturated aqueous solution), and NaCl (30 ml, saturated aqueous solution), dried over Na₂SO₄ and evaporated under reduced pressure. The crude thus obtained was purified by flash chromatography (silica gel, EtOAc/Hx), to obtain the desired product (45-63% yield).

### c.2) Synthesis of the hydroxamic acids

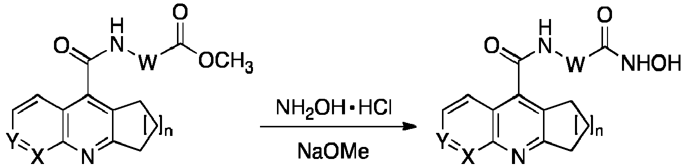

To a solution of hydroxylamine hydrochloride (3.75 mmol) and phenolphtalein (1 mg) in methanol (80 ml) under inert atmosphere, an aliquot of sodium methoxide in methanol (taken from a solution of 0.65 g, 12 mmol of sodium methoxide in 3.3 ml of methanol) was added dropwise until a permanent pink color was observed. The corresponding methyl ester (0.61 mmol) and sodium methoxide in methanol (5.0 mmol, 1.4 ml of the previously prepared solution) were subsequently added. The reaction mixture was stirred for 26h, the formation of a dense precipitate being observed. Water (3 ml) was added, and this solution was acidified with glacial acetic acid and extracted with CH₂Cl₂ (3 x 10 ml). The combined organic fractions were dried over Na₂SO₄ and evaporated under reduced pressure, to obtain the corresponding hydroxamic acid (80-95% yield).

### Method D

### d.1) Hydrolysis

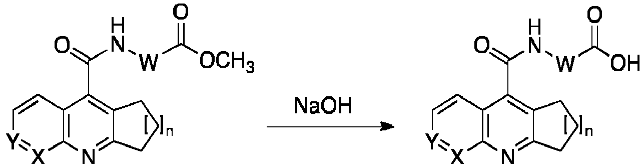

A mixture of the corresponding methyl ester (15.5 mmol), ethanol (176 ml) and 10% aqueous NaOH (85.8 ml) was refluxed for 45 minutes. Then, solvents were evaporated under reduced pressure, and the crude thus obtained was purified by flash chromatography (silica gel, EtOAc/Hx and MeOH/CH₂Cl₂) to afford the desired product (40-80% yield).

### d.2) Coupling of the benzamide chelating group

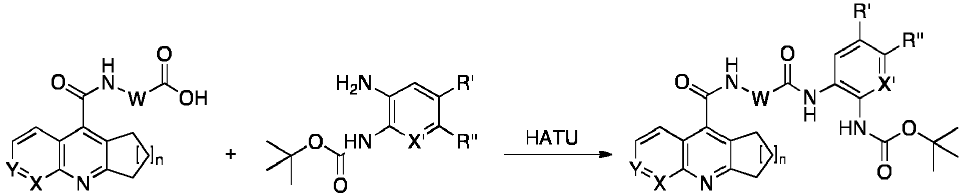

A mixture of the corresponding carboxylic acid (0.508 mmol), the corresponding tert-butyl aminecarbamate (0.508 mmol), DIPEA (0.7 ml, 4.08 mmol) and HATU (1.14 mmol) in DMF (5 ml) was stirred for 16 h under argon atmosphere. Then solvents were removed by evaporation. The residue was diluted with EtOAc and washed with water. The organic layer was dried over sodium sulfate, filtered and concentrated. The crude thus obtained was purified by flash chromatography (silica gel, 10% EtOAc/Hx) to give the desired product (45-50% yield).

### d.3) Deprotection of the carbamate moiety

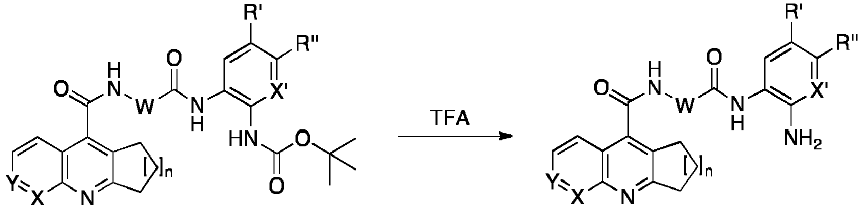

A mixture of the corresponding N-Boc protected compound (0.42 mmol), CH₂Cl₂ (3.22 ml) and TFA (1.05 ml) was stirred at room temperature for 16 hours. Then solvents were removed by evaporation. The residue was diluted with CH₂Cl₂, and washed with an ice/ NaHCO₃ mixture. The organic layer was dried over sodium sulfate, filtered and concentrated. The crude thus obtained was purified by flash chromatography (silica gel, EtOAc/Hx and MeOH/CH₂Cl₂) to obtain the desired product (50-80% yield).

### Synthesis of Compounds of the invention

### Example 1: Preparation of N-hydroxy-6-[(1,2,3,4-tetrahydroacridin-9-yl)amino]hexanamide, with the following structural formula:

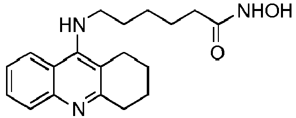

This compound was prepared following procedures described in Method A. m.p. 162-164°C; IR 3434, 3188, 3008, 1738, 1639, 1560, 1505, 1423, 1356, 1274, 1158, 754 cm⁻¹; ¹H-NMR (500 MHz, δ ppm DMSO-d₆)10.29 (s, 1H), 8.61 (s, 1H), 8.10 (d, *J* = 8.4 Hz, 1 H), 7.70 (d, *J* = 8.3 Hz, 1 H), 7.51 (t, *J* = 7.6 Hz, 1 H), 7.33 (t, *J* = 7.6 Hz, 1 H), 5.36 (s, 1 H), 3.38 (dd, *J* = 13.8 Hz, *J*' = 6.8 Hz, 2H), 2.90 (t, *J* = 6.0 Hz, 2H), 2.71 (t, *J* = 5.7 Hz, 2H), 1.91 (t, *J* = 7.3 Hz, 2H), 1.87 - 1.76 (m, 4H), 1.59 - 1.51 (m, 2H), 1.51 - 1.43 (m, 2H), 1.26 (dt, *J* = 14.6 Hz, *J*' = 7.3 Hz, 2H); ¹³C-NMR (75 MHz, δ ppm, DMSO-d₆) 169.0, 157.7, 150.5, 146.6, 128.0, 123.3, 123.1, 120.1, 115.7, 47.9, 33.3, 32.2, 30.3, 25.9, 25.0, 24.9, 22.7, 22.4. C₁₉H₂₅N₃O₂; MS (ESI, m/z): 328.22 [M+1]⁺.

### Example 2: Preparation of N-hydroxy-7-[(1,2,3,4-tetrahydroacridin-9-yl)amino]heptanamide, with the following structural formula:

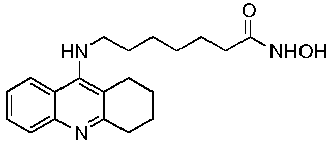

This compound was prepared following procedures described in Method A. m.p. 156-157°C; IR 3377, 3184, 1736, 1629, 1560, 1502, 1410, 1357, 1292, 1134, 765 cm⁻¹; ¹H-NMR (500 MHz, δ ppm, DMSO-d₆) 10.30 (s, 1H), 8.66 (s, 1 H), 8.10 (d, *J* = 8.4 Hz, 1 H), 7.69 (d, *J* = 8.2 Hz, 1 H), 7.51 (t, *J* = 7.6 Hz, 1 H), 7.33 (t, *J* = 7.6 Hz, 1 H), 5.34 (t, *J* = 6.4 Hz, 1 H), 3.38 (m, 2H, signal partially overlapped with broad signal of water at 3.32 ppm, confirmed by COSY), 2.90 (t, *J* = 6.0 Hz, 2H), 2.71 (t, *J* = 5.9 Hz, 2H), 1.90 (t, *J* = 7.4 Hz, 2H), 1.86 - 1.76 (m, 4H), 1.53 (dt, *J =* 15.0 Hz, *J' =* 7.4 Hz, 2H), 1.44 (dt, J = 14.9 Hz, *J'=* 7.6 Hz, 2H), 1.32 - 1.16 (m, 4H); ¹³C-NMR (126 MHz, δ ppm, DMSO-d₆) 169.0, 158.0, 150.3, 147.0, 128.4, 127.9, 123.2, 123.1, 120.3, 115.9, 48.0, 33.6, 32.2, 30.5, 28.4, 26.1, 25.1, 25.1, 22.8, 22.5. C₂₀H₂₇N₃O₂; MS (ESI, m/z): 342.17 [M+1]⁺.

### Example 3: Preparation of 7-[(2,3-dihydro-1H-cyclopenta[b]quinolin-9-yl) amino]-N-hydroxyheptanamide, with the following structural formula:

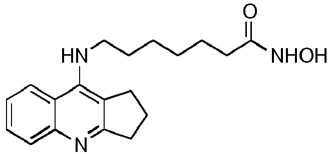

This compound was prepared following procedures described in Method A. Yield 96%; m.p. 165-166°C; IR 3371, 3184, 1737, 1631, 1543, 1416, 1364, 1026, 759 cm⁻¹; ¹H-NMR (500 MHz, δ ppm, DMSO-d₆) 10.31 (s, 1 H), 8.62 (s, 1 H), 8.21 (d, *J* = 8.4 Hz, 1 H), 7.69 (d, *J* = 8.2 Hz, 1 H), 7.55 (t, *J* = 7.4 Hz, 1 H), 7.36 (t, *J* = 7.6 Hz, 1 H), 6.81 (s, 1 H), 3.51 (dd, *J =* 13.6 Hz, *J' =* 6.6 Hz, 2H), 3.17 (t, *J =* 7.2 Hz, 2H), 2.89 (t, *J =* 7.7 Hz, 2H), 2.08 - 2.00 (m, 2H), 1.92 (t, *J* = 7.4 Hz, 2H), 1.60 - 1.53 (m, 2H), 1.51 - 1.45 (m, 2H), 1.36 - 1.31 (m, 2H), 1.29 - 1.22 (m, 2H); ¹³C-NMR (126 MHz, δ ppm, DMSO-d₆) 169.1, 166.8, 147.3, 146.4, 128.5, 126.9, 123.5, 121.9, 118.5, 111.7, 44.0, 33.7, 32.2, 30.7, 30.7, 28.4, 25.9, 25.1, 22.7. C₁₉H₂₅N₃O₂; MS (ESI, m/z): 328.35 [M+1]⁺.

### Example 4: Preparation of N-hydroxy-7-[(7,8,9,10-tetrahydro-6H-cyclohepta[b]quinolin-11-yl)amino]heptanamide, with the following structural formula:

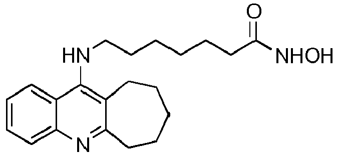

This compound was prepared following procedures described in Method A. Yield 62%; m.p. 155-156°C; IR 3319, 1738, 1632, 1501, 1447, 1350, 766 cm⁻¹; ¹H-NMR (500 MHz, δ ppm DMSO-d₆) 10.28 (s, 1H), 8.61 (s, 1 H), 8.14 (d, *J* = 8.1 Hz, 1 H), 7.74 (d, *J* = 8.4 Hz, 1 H), 7.54 (t, *J* = 7.0 Hz, 1 H), 7.40 (t, *J* = 7.0 Hz, 1 H), 5.25 (s, 1 H), 3.19 (dd, *J* = 14.0 Hz, *J' =* 6.8 Hz, 2H), 3.07 - 3.05 (m, 2H), 2.92 - 2.90 (m, 2H), 1.90 (t, *J* = 7.4 Hz, 2H), 1.86 - 1.80 (m, 2H), 1.70 - 1.63 (m, 4H), 1.58 - 1.51 (m, 2H), 1.47 - 1.41 (m, 2H), 1.31 - 1.16 (m, 4H); ¹³C-NMR (126 MHz, δ ppm, DMSO-d₆) 169.0, 164.8, 150.0, 146.3, 128.5, 127.8, 124.1, 123.1, 122.8, 122.0, 49.8, 32.2, 31.5, 30.4, 28.4, 28.1, 27.2, 26.6, 26.2, 25.1. C₂₁H₂₉N₃O₂; MS (ESI, m/z): 357.38 [M+1]⁺.

### Example 5: Preparation of N-hydroxy-7-[(5-methoxy-1,2,3,4-tetrahydroacridin -9-yl)amino]heptanamide, with the following structural formula:

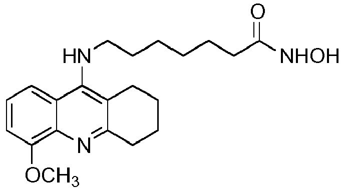

This compound was prepared following procedures described in Method A. m.p. 361°C; IR 3376, 3221, 1631, 1578, 1357, 1275, 1233, 740 cm⁻¹; ¹H-NMR (500 MHz, δ ppm, DMSO-d₆) 10.29 (s, 1H), 8.62 (s, 1H), 8.14(s, 1 H), 7.79 (d, *J* = 8.4 Hz, 1 H), 7.40 (t, *J* = 7.7 Hz, 1 H), 7.25 (s, 1 H), 3.99 (s, 3H), 3.61 (s, 2H), 2.98 (s, 2H), 2.67 (s, 2H), 1.91 (t, *J* = 7.4 Hz, 2H), 1.85 - 1.77 (m, 4H), 1.67 - 1.58 (m, 2H), 1.51 - 1.40 (m, 2H), 1.32 - 1.21 (m, 4H); ¹³C-NMR (126 MHz, δ ppm DMSO-d₆) 169.2, 164.1, 154.4, 152.5, 135.2, 124.0, 119.5, 115.2, 115.0, 108.7, 55.9, 47.8, 32.2, 31.5, 30.3, 28.4, 26.0, 25.1, 24.8, 22.3, 21.8. C₂₁H₂₉N₃O₃; MS (ESI, m/z): 372.54 [M+1]⁺.

### Example 6: Preparation of N-hydroxy-7-[(5-hydroxy-1,2,3,4-tetrahydroacridin-9-yl)amino]heptanamide, with the following structural formula:

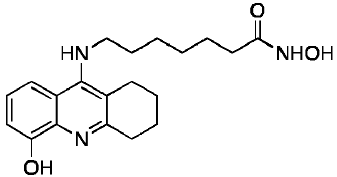

This compound was prepared following procedures described in Method A. m.p. 73°C; IR 3329, 1609, 1561, 1449, 1319, 1231, 661 cm⁻¹; ¹H-NMR (500 MHz, δ ppm, DMSO-d₆) 7.67 (d, *J* = 8.3 Hz, 1 H), 7.27 (t, *J* = 7.9 Hz, 1 H), 7.03 (d, *J* = 7.3 Hz, 1 H), 3.40 (s, 2H), 2.91 (s, 2H), 2.68 (s, 2H), 2.14 (t, *J =* 7.0 Hz, 2H), 1.79 (s, 4H), 1.53 (s, 2H), 1.43 (t, *J* = 5.8 Hz, 2H), 1.24 (s, 4H); ¹³C-NMR (126 MHz, δ ppm, DMSO-d₆) 174.5, 155.5, 153.9, 151.2, 137.3, 123.5, 120.57, 116.0, 114.8, 107.5, 55.5, 47.9, 33.7, 30.4, 28.3, 26.0, 24.9, 24.4, 22.5, 22.2. C₂₀H₂₇N₃O₃; MS (ESI, m/z): 358.23 [M+1]⁺.

### Example 7: Preparation of N-hydroxy-7-[(6,7,8,9-tetrahydrobenzo[b][1,8]naphthyridin-5-yl)amino]heptanamide, with the following structural formula:

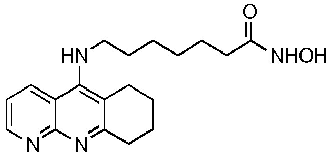

This compound was prepared following procedures described in Method A. m.p. 234-235°C; IR 3214, 1738, 1583, 1524, 1440, 1349, 1234, 774 cm⁻¹; ¹H-NMR (500 MHz, δ ppm, DMSO-d₆) 10.29 (s, 1 H), 8.81 (d, *J* = 4.2Hz, 1 H), 8.55 (d, *J* = 8.5 Hz, 1 H), 8.48 - 8.42 (m, 1 H), 7.33 (dd, *J* = 8.3Hz, *J'* = 4.2 Hz, 1 H), 5.67 (t, *J* = 5.6 Hz, 1 H), signal corresponding to 2H overlapped with broad signal of water at 3.42 ppm (confirmed by COSY), 2.92 (t, *J* = 5.9 Hz, 2H), 2.69 (t, *J* = 5.7 Hz, 2H), 1.89 (t, *J* = 7.4 Hz, 2H), 1.85 - 1.79 (m, 4H), 1.57 - 1.52 (m, 2H), 1.50 - 1.39 (m, 4H), 1.31 - 1.16 (m, 2H); ¹³C-NMR (126 MHz, δ ppm, DMSO-d₆) 169.1, 160.8, 154.8, 151.8, 151.4, 133.1, 118.6, 115.5, 113.9, 47.9, 33.8, 32.2, 30.4, 28.4, 26.1, 25.1, 25.0, 22.6, 22.3. C₁₉H₂₆N₄O₂; MS (ESI, m/z): 343.34 [M+1]⁺.

### Example 8: Preparation of N-hydroxy-7-[(6,7,8,9-tetrahydrobenzo[b][1,7]naphthyridin-5-yl)amino]heptanamide, with the following structural formula:

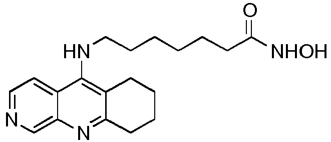

This compound was prepared following procedures described in Method A. Yield 70%; m.p. 56.7°C; IR 3409, 3221, 1726, 1627, 1543, 1501, 1418, 1262, 1159 cm⁻¹; ¹H-NMR (500 MHz, δ ppm, DMSO-d₆) 10.28 (s, 2H), 9.03 (s, 1 H), 8.61 (s, 1 H), 8.32 (d, *J =* 5.7 Hz, 1 H), 7.97 (d, *J* = 5.8 Hz, 1 H), 3.48 (dd, J = 13.8, 6.9 Hz, 2H), 2.94 (t, *J* = 6.2 Hz, 2H), 2.70 (t, *J* = 6.0 Hz, 2H), 1.93 - 1.86 (m, 2H), 1.85 - 1.77 (m, 2H), 1.60 - 1.51 (m, 2H), 1.49 - 1.41 (m, 2H), 1.31 - 1.19 (m, 6H); ¹³C-NMR (126 MHz, δ ppm, DMSO-d₆) 169.1, 159.7, 152.8, 148.9, 142.2, 140.2, 122.6, 117.8, 115.9, 47.2, 33.6, 32.2, 30.4, 28.3, 25.9, 25.3, 25.1, 22.4, 22.2. C₁₉H₂₆N₄O₂- MS (ESI, m/z): 343.34 [M+1]⁺.

### Example 9: Preparation of N-hydroxy-4-{[(1,2,3,4-tetrahydroacridin-9-yl)amino]methyl}benzamide, with the following structural formula:

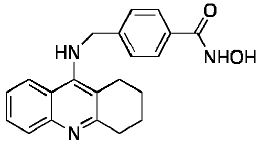

This compound was prepared following procedures described in Method A. m.p. 204-205°C; IR 3426, 3378, 3222, 1738, 1631, 1570, 1503, 1438, 1351, 1292, 1136, 758, 617 cm⁻¹; ¹H-NMR (500 MHz, δ ppm, DMSO-d₆) 8.96 (s, 2H), 8.09 (d, *J* = 8.5 Hz, 1 H), 7.70 (d, *J* = 8.3 Hz, 1 H), 7.67 (d, *J* = 8.1 Hz, 2H), 7.52 (t, *J* = 7.5 Hz, 1 H), 7.41 (d, *J* = 8.1 Hz, 2H), 7.30 (t, *J* = 7.7 Hz, 1 H), 6.27 (s, 1 H), 4.68 (d, *J* = 6.1 Hz, 2H), 2.89 (t, *J* = 6.2 Hz, 2H), 2.72 (t, *J* = 6.0 Hz, 2H), 1.95 - 1.62 (m, 4H); ¹³C-NMR (126 MHz, δ ppm, DMSO-d₆) 164.0, 157.4, 150.4, 146.0, 143.8, 131.4, 129.5, 128.4, 127.5, 127.0, 126.9, 123.6, 123.0, 119.7, 115.8, 50.5, 32.9, 25.1, 22.6, 22.2. C₂₁H₂₁N₃O₂; MS (ESI, m/z): 348.17 [M+1]⁺.

### Example 10: Preparation of N-[6-(hydroxyamino)-6-oxohexyl]-1,2,3,4-tetrahydroacridine-9-carboxamide, with the following structural formula:

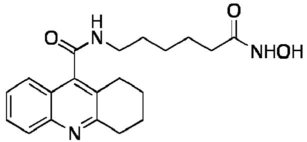

This compound was prepared following procedures described in Method C. m.p. 187-188°C; IR cm⁻¹ 3258, 1666, 1627, 1535, 1495, 1428, 1358, 1263, 1169, 764 cm⁻¹; ¹H-NMR (500 MHz, δ ppm, DMSO-d₆) 10.35 (s, 1 H), 8.68 - 8.60 (m, 2H), 7.90 (d, *J* = 8.4 Hz, 1 H), 7.69 - 7.66 (m, 1 H), 7.64 (d, *J* = 7.5 Hz, 1 H), 7.53 (t, *J* = 7.5 Hz, 1 H), signal corresponding to 2H overlapped with broad signal of water at 3.35 ppm (confirmed by COSY), 3.04 (t, *J* = 6.4 Hz, 2H), 2.83 (t, *J* = 6.1 Hz, 2H), 1.97 (t, *J* = 7.4 Hz, 2H), 1.92 - 1.88 (m, 2H), 1.85 - 1.81 (m, 2H), 1.61 - 1.50 (m, 4H), 1.41 - 1.30 (m, 2H); ¹³C-NMR (126 MHz, δ ppm, DMSO-d₆) 169.0, 166.3, 158.8, 145.7, 142.2, 128.7, 128.2, 126.1, 125.8, 124.6, 123.1, 38.6, 33.4, 32.2, 28.7, 26.1, 25.9, 24.8, 22.4, 22.1. C₂₀H₂₅N₃O₃; MS (ESI, m/z): 356.13 [M+1]⁺.

### Example 11: Preparation of N-[7-(hydroxyamino)-7-oxoheptyl]-1,2,3,4-tetrahydroacridine-9-carboxamide, with the following structural formula:

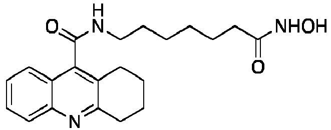

This compound was prepared following procedures described in Method C. m.p. 185-186°C; IR 3404, 3265, 3163, 1634, 1540, 1407, 754 cm⁻¹; ¹H-NMR (500 MHz, δ ppm, DMSO-d₆) 10.35 (s, 1 H), 8.68 - 8.61 (m, 2H), 7.90 (d, *J* = 8.5 Hz, 1 H), 7.68 - 7.63 (m, 2H), 7.52 (t, *J* = 7.6 Hz, 1 H), signal corresponding to 2H overlapped with broad signal of water at 3.39 ppm (confirmed by COSY), 3.03 (t, *J* = 5.9 Hz, 2H), 2.84 (t, *J* = 5.8 Hz, 2H), 1.96 (t, *J* = 7.0 Hz, 2H), 1.93 - 1.87 (m, 2H), 1.85 - 1.79 (m, 2H), 1.61 - 1.46 (m, 4H), 1.38 - 1.27 (m, 4H); ¹³C-NMR (126 MHz, δ ppm, DMSO-d₆) 169.1, 166.3, 158.8, 145.7, 142.2, 128.8, 128.2, 126.1, 125.8, 124.6, 123.2, 38.7, 33.4, 32.3, 28.9, 26.3, 25.9, 25.2, 24.9, 22.4, 22.1. C₂₁H₂₇N₃O₃; MS (ESI, m/z): 370.13 [M+1]⁺.

### Example 12: Preparation of N-(2-amino-4-methylphenyl)-7-[(1,2,3,4-tetrahydroacridin-9-yl)amino]heptanamide, with the following structural formula:

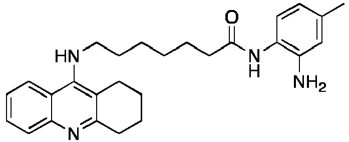

This compound was prepared following procedures described in Method B. m.p. 63-64°C; IR 3326, 3174, 3061, 1719, 1651, 1568, 1379, 1227, 1091, 712, 621 cm-¹; ¹H-NMR (500 MHz, δ ppm, DMSO-d₆) 8.98 (s, 1 H), 8.20 (d, *J* = 8.6 Hz, 1 H), 7.74 (d, *J* = 8.3 Hz, 1 H), 7.62 (t, *J* = 7.3 Hz, 1 H), 7.41 (t, *J* = 7.4 Hz, 1 H), 6.98 (d, *J* = 7.9 Hz, 1 H), 6.51 (s, 1 H), 6.33 (d, *J* = 7.5 Hz, 1 H), 6.11 (s, 1 H), 4.70 (s, 2H), 3.54 (s, 2H), 2.92 (t, *J* = 5.2 Hz, 2H), 2.69 (t, *J* = 5.3 Hz, 2H), 2.26 (t, *J* = 7.3 Hz, 2H), 2.14 (s, 3H), 1.88 - 1.75 (m, 4H), 1.66 - 1.59 (m, 2H), 1.59 - 1.52 (m, 2H), 1.40 - 1.27 (m, 4H); ¹³C-NMR (126 MHz, δ ppm, DMSO-d₆) 171.1, 155.4, 152.1, 143.9, 141.8, 134.7, 129.5, 125.3, 125.2, 123.8, 123.7, 121.2, 118.7, 116.9, 116.3, 114.3, 47.8, 35.6, 31.7, 30.3, 28.4, 26.1, 25.2, 24.7, 22.3, 21.8, 20.8. C₂₇H₃₄N₄O. MS (ESI, m/z): 431.22 [M+1]⁺.

### Example 13: Preparation of N-(2-amino-5-methylphenyl)-7-((1,2,3,4-tetrahydroacridin-9-yl)amino)heptanamide, with the following structural formula:

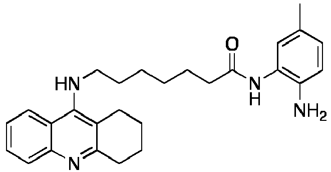

This compound was prepared following procedures described in Method B. m.p. 204-205°C; IR 3242, 1682, 1574, 1517, 1415, 1359, 1199, 1127, 758 cm⁻¹; ¹H-NMR (500 MHz, δ ppm, DMSO-d₆) 9.02 (s, 1 H), 8.19 (d, *J* = 8.3 Hz, 1 H), 7.73 (d, *J* = 8.3 Hz, 1 H), 7.61 (t, *J* = 7.4 Hz, 1 H), 7.40 (t, *J* = 7.4 Hz, 1 H), 6.96 (s, 1 H), 6.70 (d, *J* = 7.9 Hz, 1 H), 6.61 (d, *J* = 8.0 Hz, 1 H), 6.06 (s, 1 H), 4.58 (s, 2H), 3.62 - 3.49 (m, 2H), 2.92 (t, *J* = 5.3 Hz, 2H), 2.69 (t, *J* = 5.2 Hz, 2H), 2.27 (t, *J* = 7.2 Hz, 2H), 2.12 (s, 3H), 1.81 (d, *J* = 4.7 Hz, 4H), 1.66 - 1.51 (m, 4H), 1.37 - 1.28 (m, 4H); ¹³C-NMR (126 MHz, δ ppm, DMSO-d₆) 171.0, 155.7, 151.9, 144.3, 139.2, 129.3, 126.2, 125.5, 124.7, 123.8, 123.7, 123.6, 118.9, 116.1, 114.5, 47.8, 35.7, 31.9, 30.3, 28.4, 26.1, 25.3, 24.8, 22.4, 21.8, 20.1. C₂₇H₃₄N₄O. MS (ESI, m/z): 431.22 [M+1]⁺.

### Example 14: Preparation of N-[2-amino-5-(tert-butyl)phenyl]-7-[(1,2,3,4-tetrahydroacridin-9-yl)amino]heptanamide, with the following structural formula:

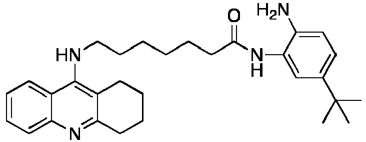

This compound was prepared following procedures described in Method B. m.p. 75-76°C; IR 3233, 1648, 1499, 1419, 1358, 818, 758 cm⁻¹; ¹H-NMR (500 MHz, δ ppm, DMSO-d₆) 9.13 (s, 1 H), 8.11 (d, *J =* 8.4 Hz, 1 H), 7.70 (d, *J =* 8.4 Hz, 1 H), 7.51 (t, *J =* 7.5 Hz, 1 H), 7.33 (t, *J* = 7.5 Hz, 1 H), 7.11 (d, *J* = 1.8 Hz, 1 H), 6.93 (dd, *J* = 8.2 Hz, *J' =* 1.9 Hz, 1 H), 6.65 (d, *J* = 8.3 Hz, 1 H), 5.40 (s, 1 H), 4.59 (s, 2H), signal corresponding to 2H overlapped with broad signal of water at 3.36 ppm (confirmed by COSY), 2.89 (t, *J* = 6.1 Hz, 2H), 2.71 (t, *J* = 5.8 Hz, 2H), 2.26 (t, *J* = 7.3 Hz, 2H), 1.84 - 1.76 (m, 4H), 1.56 (dd, *J=* 13.9 Hz, *J'=* 6.8 Hz, 4H), 1.34 - 1.28 (s, 4H), 1.19 (s, 9H); ¹³C-NMR (126 MHz, δ ppm, DMSO-d₆) 171.1, 157.9, 150.5, 146.8, 139.4, 138.8, 128.2, 127.9, 123.3, 123.3, 123.1, 122.6, 121.8, 120.2, 115.9, 115.8, 48.0, 35.7, 33.5, 31.4, 30.6, 28.5, 26.2, 25.3, 25.1, 22.8, 22.5. C₃₀H₄₀N₄O. MS (ESI, m/z): 473.23 [M+1]⁺.

### Example 15: Preparation of N-(4-amino-[1,1'-biphenyl]-3-yl)-7-[(1,2,3,4-tetrahydroacridin-9-yl)amino]heptanamide, with the following structural formula:

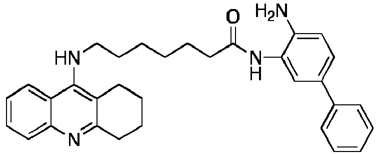

This compound was prepared following procedures described in Method B. m.p. 101-102°C; IR 3341, 3219, 1667, 1642, 1563, 1516, 1412, 1359, 1199, 1127, 759, 698 cm⁻¹; ¹H-NMR (500 MHz, δ ppm, DMSO-d₆) 9.18 (s, 1 H), 8.38 (d, *J* = 8.6 Hz, 1 H), 7.88 - 7.79 (m, 2H), 7.72 - 7.61 (m, 1 H), 7.56 (t, *J* = 8.3 Hz, 1 H), 7.53 (d, *J* = 2.0 Hz, 1H), 7.49 (d, *J* = 7.3 Hz, 2H), 7.37 (t, *J* = 7.7 Hz, 2H), 7.25 - 7.20 (m, 2H), 6.79 (d, *J* = 8.3 Hz, 1 H), 5.02 (s, 2H), 3.85 (d, J = 6.5 Hz, 2H), 2.97 (s, 2H), 2.66 (s, 2H), 2.34 (t, J = 7.3 Hz, 2H), 1.82 (s, 4H), 1.78 - 1.71 (m, 2H), 1.66 - 1.56 (m, 2H), 1.38 (s, 4H); ¹³C-NMR (126 MHz, δ ppm, DMSO-d₆) 171.4, 155.7, 150.6, 141.4, 140.4, 138.0, 132.7, 128.9, 128.8, 128.0, 126.9, 126.0, 125.5, 125.1, 125.0, 123.9, 123.8, 123.2, 119.3, 116.3, 115.5, 111.2, 47.3, 35.7, 29.8, 29.6, 28.3, 27.9, 25.9, 25.2, 23.9, 21.5. C₃₂H₃₄N₄O. MS (ESI, m/z): 493.46 [M+1]⁺.

### Example 16: Preparation of N-(4-Amino-[1,1'-biphenyl]-3-yl)-6-[(1,2,3,4-tetrahydroacridin-9-yl)amino] hexanamide, with the following structural formula:

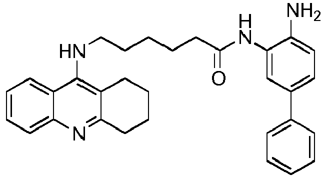

This compound was prepared following procedures described in Method B. m.p. 75-76°C; IR 3341, 3235, 1670, 1635, 1574, 1517, 1414, 1359, 1199, 1127, 758, 698 cm⁻¹; ¹H-NMR (500 MHz, δ ppm, DMSO-d₆) 9.12 (s, 1H), 8.30 (t, *J* = 9.2 Hz, 1H), 7.76 (s, 2H), 7.66 (d, J = 7.9 Hz, 2H), 7.54 - 7.43 (m, 3H), 7.37 (t, 2H), 7.23 (s, 2H), 6.80 (d, *J* = 7.7 Hz, 1 H), 4.99 (s, 2H), 3.73 (s, 2H), 2.93 (s, 2H), 2.63 (s, 2H), 2.35 (d, *J* = 7.1 Hz, 2H), 1.79 (s, 4H), 1.74 (s, 2H), 1.45 - 1.34 (m, 4H); ¹³C-NMR (126 MHz, δ ppm, DMSO-d₆) 171.2, 155.8, 150.5, 141.2, 140.4, 137.9, 132.7, 129.7, 128.9, 128.8, 126.9, 126.8, 126.0, 125.5, 125.2, 125.1, 123.9, 123.2, 119.1, 116.2, 115.5, 111.2, 47.2, 35.6, 29.5, 29.1, 27.9, 26.9, 25.6, 21.4, 20.3. C₃₁H₃₄N₄O. MS (ESI, m/z): 479.43 [M+1]⁺.

### Example 17: Preparation of N-(4-amino-3'-methyl-[1,1'-biphenyl]-3-yl)-7-[(1,2,3,4-tetrahydroacridin-9-yl)amino]heptanamide, with the following structural formula:

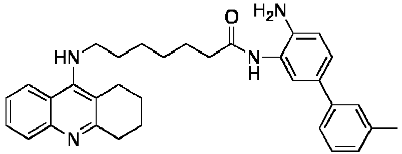

This compound was prepared following procedures described in Method B. m.p. 53-54°C; IR 3264, 1742, 1635, 1566, 1516, 1410, 1198, 752 cm⁻¹; ¹H-NMR (500 MHz, δ ppm, DMSO-d₆) 9.12 (s, 1 H), 8.12 (d, *J* = 7.9 Hz, 2H), 7.70 (d, *J* = 8.2 Hz, 2H), 7.52 (s, 1 H), 7.49 (s, 1 H), 7.38 - 7.19 (m, 6H), 7.04 (d, *J* = 6.9 Hz, 1 H), 6.78 (d, *J* = 8.2 Hz, 1 H), signal corresponding to 2H overlapped with broad signal of water at 3.42 ppm (confirmed by COSY), 2.90 (d, *J* = 4.2 Hz, 2H), 2.70 (s, 2H), 1.90 (s, 3H), 1.80 (s, 4H), 1.58 (s, 4H), 1.35 - 1.20 (m, 6H); ¹³C-NMR (126 MHz, δ ppm, DMSO-d₆) 171.4, 157.2, 150.51, 141.4, 140.3, 137.8, 130.0, 128.7, 128.3, 127.5, 127.0, 126.7, 126.2, 123.9, 123.9, 123.4, 123.3, 122.7, 119.8, 117.9, 117.8, 116.3, 47.9, 35.8, 30.5, 28.5, 26.2, 25.2, 25.0, 24.6, 22.7, 22.3, 21.2. C₃₃H₃₈N₄O. MS (ESI, m/z): 507.33 [M+1]⁺.

### Example 18: Preparation of N-{4-amino-4'-(tert-butyl)-[1,1'-biphenyl]-3-yl}-7-[(1,2,3,4-tetrahydroacridin-9-yl)amino]heptanamide, with the following structural formula:

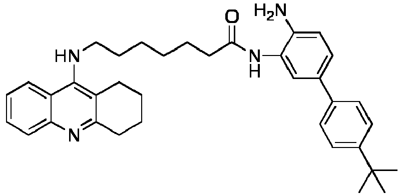

This compound was prepared following procedures described in Method B. m.p. 55-56°C; IR 3247, 1726, 1643, 1561, 1495, 1414, 1267, 1121, 817, 756, 558 cm⁻¹; ¹H-NMR (500 MHz, δ ppm, DMSO-d₆) 9.12 (s, 1H), 8.10 (t, *J* = 8.4 Hz, 1H), 7.69 (d, *J* = 8.8 Hz, 2H), 7.53 - 7.47 (m, 2H), 7.40 (dd, *J* = 18.5 Hz, J' = 8.3 Hz, 4H), 7.32 (t, *J* = 7.6 Hz, 1H), 7.20 (d, *J* = 7.3 Hz, 1 H), 6.78 (d, *J* = 8.3 Hz, 1 H), 4.94 (s, 2H), signal corresponding to 2H overlapped with broad signal of water at 3.42 ppm (confirmed by COSY), 2.89 (t, *J =* 6.7 Hz, 2H), 2.71 (dd, *J =* 12.8, 7.0 Hz, 2H), 1.85 - 1.74 (m, 4H), 1.63 - 1.50 (m, 4H), 1.39 - 1.17 (m, 15H); ¹³C-NMR (126 MHz, δ ppm, DMSO-d₆) 171.2, 157.9, 150.3, 148.3, 146.9, 141.2, 137.5, 131.6, 128.6, 128.3, 127.8, 125.5, 125.2, 123.9, 123.7, 123.1, 123.0, 120.3, 116.2, 115.9, 47.9, 38.1, 33.5, 31.1, 30.5, 29.8, 28.3, 26.1, 25.1, 23.2, 22.7, 22.4. C₃₆H₄₄N₄O. MS (ESI, m/z): 549.37 [M+1]⁺.

### Example 19: Preparation of N-[2-amino-5-(naphthalen-2-yl)phenyl]-7-[(1,2,3,4-tetrahydroacridin-9-yl)amino]heptanamide, with the following structural formula:

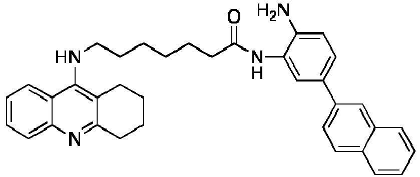

This compound was prepared following procedures described in Method B. m.p. 100-101°C; IR 3232, 1668, 1573, 1415, 1198, 1172, 1124, 813, 751, 719 cm⁻¹; ¹H-NMR (500 MHz, δ ppm, DMSO-d₆) 9.22 (s, 1 H), 8.36 (d, *J =* 8.6 Hz, 1 H), 8.00 (s, 1 H), 7.91 (d, *J* = 8.4 Hz, 2H), 7.88 (d, *J* = 7.6 Hz, 1 H), 7.82 - 7.77 (m, 2H), 7.71 (dd, *J* = 8.5, 1.1 Hz, 1 H), 7.68 (d, *J* = 1.8 Hz, 1 H), 7.57 - 7.51 (m, 2H), 7.51 - 7.47 (m, 1 H), 7.46 - 7.43 (m, 1 H), 7.40 (dd, *J* = 8.3 Hz, *J' =* 2.0 Hz, 1 H), 6.85 (d, *J* = 8.3 Hz, 1 H), 5.08 (s, 2H), 3.85 - 3.76 (m, 2H), 2.99 - 2.91 (m, 2H), 2.68 - 2.62 (m, 2H), 2.36 (t, *J* = 7.3 Hz, 2H), 1.85 - 1.79 (m, 2H), 1.77 - 1.70 (m, 2H), 1.67 - 1.56 (m, 2H), 1.41 - 1.34 (m, 4H), 1.23 (s, 2H); ¹³C-NMR (126 MHz, δ ppm, DMSO-d₆) 171.4, 141.7, 137.7, 133.5, 131.6, 128.3, 127.8, 127.7, 127.5, 126.3, 125.4, 124.9, 124.6, 124.3, 123.9, 123.6, 123.2, 116.3, 47.4, 35.7, 29.8, 29.0, 28.3, 25.9, 25.1, 24.0, 21.6, 20.6. C₃₆H₃₈N₄O. MS (ESI, m/z): 543.19 [M+1]⁺.

### Example 20: Preparation of N-(2-amino-5-phenylpyridin-3-yl)-7-[(1,2,3,4-tetrahydroacridin-9-yl)amino]heptanamide, with the following structural formula:

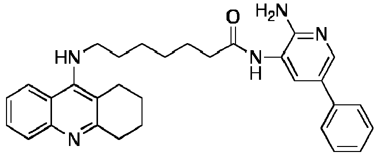

This compound was prepared following procedures described in Method B. m.p. 49-50°C; IR 3318, 3222, 1648, 1561, 1498, 1465, 1409, 758, 696 cm⁻¹; ¹H-NMR (500 MHz, δ ppm, DMSO-d₆) 9.13 (s, 1 H), 8.13 - 8.09 (m, 2H), 7.97 (d, *J* = 2.0 Hz, 1 H), 7.70 (d, *J* = 8.3 Hz, 1 H), 7.55 - 7.49 (m, 3H), 7.41 (t, *J* = 7.7 Hz, 2H), 7.33 (t, *J* = 7.5 Hz, 1 H), 7.28 (t, *J* = 7.3 Hz, 1 H), 5.91 (s, 2H), 5.45 (s, 1 H), signal corresponding to 2H overlapped with broad signal of water at 3.39 ppm (confirmed by COSY), 2.89 (t, *J* = 6.1 Hz, 2H), 2.70 (t, *J =* 5.5 Hz, 2H), 2.33 (t, *J =* 7.4 Hz, 2H), 1.80 (dd, *J =* 12.5 Hz, *J' =* 8.3 Hz, 4H), 1.62 - 1.51 (m, 4H), 1.38 - 1.26 (m, 4H); ¹³C-NMR (126 MHz, δ ppm, DMSO-d₆) 171.9, 157.7, 153.8, 153.2, 152.4, 152.2, 141.5, 141.1, 137.8, 131.0, 128.9, 126.6, 125.6, 124.5, 124.4, 122.5, 118.8, 117.2, 112.9, 47.5, 35.8, 30.0, 28.3, 26.0, 25.0, 24.3, 21.9, 21.1. C₃₁H₃₅N₅O. MS (ESI, m/z): 494.16 [M+1]⁺.

### Example 21: Preparation of N-[2-amino-5-(pyridin-3-yl)phenyl]-7-[(1,2,3,4-tetrahydroacridin-9-yl)amino]heptanamide, with the following structural formula:

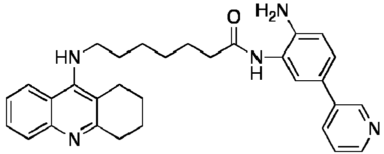

This compound was prepared following procedures described in Method B. m.p. 95-96°C; IR 3363, 3221, 1637, 1561, 1499, 1465, 1411, 1199, 759, 695 cm⁻¹; ¹H-NMR (500 MHz, δ ppm, DMSO-d₆) 9.18 (s, 1 H), 8.73 (s, 1 H), 8.43 (s, 1 H), 8.11 (d, *J =* 8.4 Hz, 1 H), 7.88 (d, *J* = 7.9 Hz, 1 H), 7.70 (d, *J* = 8.4 Hz, 1 H), 7.56 (d, *J* = 1.8 Hz, 1 H), 7.51 (t, *J* = 7.5 Hz, 1 H), 7.39 (dd, *J* = 7.7 Hz, *J' =* 4.8 Hz, 1 H), 7.33 (t, *J* = 7.6 Hz, 1 H), 7.29 (dd, *J =* 8.3 Hz, *J' =* 1.8 Hz, 1 H), 6.82 (d, *J =* 8.3 Hz, 1 H), 5.46 (s, 1 H), 5.12 (s, 2H), signal corresponding to 2H overlapped with broad signal of water at 3.39 ppm (confirmed by COSY), 2.89 (t, J = 5.7 Hz, 2H), 2.70 (t, J = 5.9 Hz, 2H), 2.31 (t, J = 7.4 Hz, 2H), 1.86 - 1.72 (m, 4H), 1.64 - 1.51 (m, 4H), 1.32 (d, J = 3.0 Hz, 4H); ¹³C-NMR (126 MHz, δ ppm, DMSO-d₆) 171.4, 157.9, 150.3, 147.0, 146.9, 146.7, 142.1, 135.7, 132.7, 128.4, 127.8, 124.6, 124.1, 123.9, 123.8, 123.4, 123.2, 123.1, 120.3, 116.3, 115.9, 47.9, 35.8, 33.6, 30.5, 28.5, 26.2, 25.2, 25.1, 22.8, 22.5. C₃₁H₃₅N₅O. MS (ESI, m/z): 494.27 [M+1]⁺.

### Biological Assays

**Example 21:** *In vitro inhibitory activity* of *histone deacetylase: human isoforms HDAC1,* 2, 3, 4, 5, 6, 7, 8, 9, *10 and 11 and HeLa cell line nuclear extract (IC50 data).*

### Components of Assay

*Substrate peptides*: All HDAC assays were performed using acetylated AMC-labeled peptide substrate:
- Substrate for isoforms HDAC1, 2, 3, 6, 10, 11 and HeLa nuclear extract assays: Acetylated fluorogenic peptide from p53 residues 379-382 (RHKKAc).
- Substrate for isoforms HDAC 4, 5, 7, 9: Fluorogenic Boc-L-Lys(ε-trifluoroacetyl)-AMC.
- Substrate for HDAC8 assays: Acetylated fluorogenic peptide from p53 residues 379-382 (RHKAcKAc).

*Assay buffer*: 50 mM Tris-HCI, pH 8.0, 137 mM NaCl, 2.7 mM KCI, 1 mM MgCl₂ (supplement with 1 mg/ml BSA for dilution) (BioMol Cat. # KI-143).

### Enzymes:

- HDAC1 assay: 75 nM Human HDAC1 (GenBank Accession No. NM_004964): Full length with C-terminal GST tag, MW= 79.9 kDa, expressed by baculovirus expression system in Sf9 cells (BioMol Cat. # SE-456).
- HDAC2 assay: 5 nM Human HDAC2 (GenBank Accession No. Q92769): Full length with C-terminal His tag, MW= 60 kDa, expressed by baculovirus expression system in Sf9 cells (BioMol Cat. # SE-500).
- HDAC3 assay: 2.3 nM Human HDAC3/NcoR2 (GenBank Accession No. NM_003883 for HDAC3, GenBank Accession No.NM_006312 for NcoR2): Complex of human HDAC3, full length with C-terminal His tag, MW= 49.7 kDa, and human NCOR2, N-terminal GST tag, MW= 39 kDa, co-expressed in baculovirus expression system (BioMol Cat. # SE-507).
- HDAC4 assay: 266 nM Human HDAC4 (GenBank Accession No. NM_006037): Amino acids 627-1085 with N-terminal GST tag, MW= 75.2 kDa, expressed in baculovirus expression system (BioMol, Hamburg, Germany).
- HDAC5 assay: 588 nM Human HDAC5 (GenBank Accession No. NM_001015053): Full length with Nterminal GST tag, MW= 150 kDa, expressed by baculovirus expression system in Sf9 cells (BioMol, Hamburg, Germany).
- HDAC6 assay: 13 nM Human HDAC6 (GenBank Accession No. BC069243): Full length with N-terminal GST tag, MW= 159 kDa, expressed by baculovirus expression system in Sf9 cells (BioMol Cat. # SE-508).
- HDAC7 assay: 962 nM Human HDAC7 (GenBank Accession No. AY302468): Amino acids 518-end with N-terminal GST tag, MW= 78 kDa, expressed in baculovirus expression system (BioMol, Hamburg, Germany).
- HDAC8 assay: 119 nM Human HDAC8 (GenBank Accession No. NM018486): Full length, MW=42 kDa, expressed in an E. coli expression system (BioMol Cat. # SE-145).
- HDAC9 assay: 986 nM Human HDAC9 (GenBank Accession No. NM178423): Amino acids 604-1066 with C-terminal His tag, MW= 50.7 kDa, expressed in baculovirus expression system (BioMol, Hamburg, Germany).
- HDAC10 assay: 781 nM Human HDAC10 (GenBank Accession No. NM_032019): Amino acids 1-631 with Nterminal GST tag, MW= 96 kDa, expressed by baculovirus expression system in Sf9 cells (BioMol Cat. # SE-559).
- HDAC11 assay: 781 nM Human HDAC11 (GenBank Accession No. NM_BC009676) with N-terminal GST tag, MW= 66 kDa, expressed in baculovirus expression system (BioMol Cat. # SE-560).
- HeLaNuclear Extract assay: 25 ng/µl Nuclear Extract from HeLa Cells: Prepared by high salt extraction of HeLa nuclei (human cervical cancer cell line), this extract is a rich source of HDAC activity (BioMol Cat. # KI-140).

### Assay procedure

50 µM of substrate peptide (see 'substrate peptides' section above) and an optimal concentration of the corresponding enzyme (see 'enzymes' section above) in the assay buffer and 1% final concentration of DMSO were incubated in the presence of gradient concentrations of inhibitors (10-dose IC50 mode with 3-fold serial dilution) at 30°C for 2 h. The reactions were carried out in a 96-well microplate for fluorometry in a 50µl reaction volume. After the deacetylation reaction, Fluor-de-Lys-Developer (BioMol Cat. # KI-105) was added to each well to digest the deacetylated substrate, thus producing the fluorescent signal. The reaction was allowed to develop for 45 minutes at 30°C with 5% CO₂; then the fluorescent signal was measured with an excitation wavelength at 360 nm and an emission wavelength at 460 nm in a microplate-reading fluorometer (GeminiXS; Molecular Devices, Sunnyvale, CA). A curve of Deacetylated Standard (Biomol, Cat. # KI-142; made from 100 µM with 1:2 dilution and 10-doses, 6 µl) allowed the conversion of fluorescent signal into micromoles of deacetylated product. All experiments were performed in triplicate. IC50 was calculated by fitting the experimental data to a dose-response curve. DMSO was used as negative control; Trichostatin A (Biomol Cat. # GR-309) was used as positive control inhibitor.

| | **IC50 (µM)** | | | | | | | | | |
|---|---|---|---|---|---|---|---|---|---|---|
| | **Example 1** | **Example 2** | **Example 3** | **Example 4** | **Example 5** | **Example 7** | **Example 8** | **Example 9** | **Example 10** | **Example 11** |
| **HDAC-1** | 2.99 | 0.36 | 0.27 | 0.84 | 0.90 | 1.77 | 1.12 | ND | > 5 | 2.95 |
| **HDAC-2** | 6.09 | 0.75 | 0.77 | 1.92 | 1.84 | 4.46 | 2.41 | ND | > 5 | 8.48 |
| **HDAC-3** | 2.84 | 0.42 | 0.36 | 0.74 | 0.66 | 2.44 | 1.88 | ND | > 5 | 3.62 |
| **HDAC-4** | 6.19 | > 5 | > 5 | > 5 | > 5 | > 5 | > 5 | ND | > 5 | > 5 |
| **HDAC-5** | 2.14 | 0.44 | > 5 | > 5 | > 5 | > 5 | > 5 | ND | > 5 | 4.24 |
| **HDAC-6** | 0.036 | 0.01 | 0007 | 0.015 | 0.015 | 0.038 | 0.033 | ND | 0.33 | 0.19 |
| **HDAC-7** | 4.55 | 0.77 | > 5 | > 5 | > 5 | > 5 | > 5 | ND | > 5 | > 5 |
| **HDAC-8** | 0.59 | 0.53 | 1.28 | 0.84 | 1.52 | 1.44 | 1.09 | ND | 020 | 0.21 |
| **HDAC-9** | 2.47 | 1.57 | > 5 | > 5 | > 5 | > 5 | > 5 | ND | 0035 | 3.17 |
| **HDAC-10** | 5.76 | 0.71 | 0.51 | 1.13 | 1.46 | 4.02 | 2.02 | ND | > 5 | 6.89 |
| **HDAC-11** | 2.21 | 0.59 | 0.44 | 1.22 | 1.04 | 0.69 | 0.60 | ND | > 5 | 4.10 |
| **HeLa** | 0.066 | 0.023 | 0.019 | 0.038 | 0.033 | 0.12 | 0.095 | 0.90 | 0.85 | 0.42 |

| | | | | | | | | | | |
|---|---|---|---|---|---|---|---|---|---|---|
| ND: not determined | | | | | | | | | | |

| | | **IC50(µM)** | | | | | | | | |
|---|---|---|---|---|---|---|---|---|---|---|
| | | **Example 12** | **Example 13** | **Example 14** | **Example 15** | **Example 17** | **Example 18** | **Example 19** | **Example 20** | **Example 21** |
| **No preincubation** | **HDAC-1** | > 50 | 57.3 | > 50 | 1.26 | 250 | 46.7 | > 50 | 594 | 3.97 |
| | **HDAC-2** | > 50 | 62.9 | > 50 | 1.70 | 188 | > 50 | > 50 | > 50 | 3.37 |
| | **HDAC-6** | > 50 | > 50 | ND | 13.15 | ND | ND | ND | ND | ND |
| | **HeLa** | ND | ND | > 50 | 4.68 | 84.1 | 429 | 17.7 | > 50 | 78.1 |
| **4h preincubation** | **HDAC-1** | ND | > 50 | > 50 | 1.48 | 118 | 48.1 | 139 | > 50 | 8.55 |
| | **HDAC-2** | ND | 54.3 | > 50 | 0.079 | 76.0 | 65.9 | 72.7 | > 50 | 2.79 |
| | **HDAC-6** | ND | ND | ND | ND | ND | ND | ND | ND | ND |
| | **HeLa** | ND | ND | > 50 | 3.99 | 73.6 | 25.5 | 39.3 | 109 | 30.7 |

| | | | | | | | | | | |
|---|---|---|---|---|---|---|---|---|---|---|
| ND: not determined | | | | | | | | | | |

## Claims

1. A compound of general formula (I), 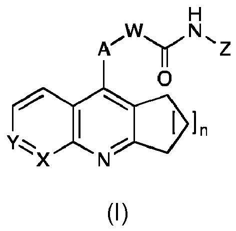 wherein:
X and Y are independently selected from a N atom or a C-R group, wherein R is selected from a hydrogen atom, an alkyl, an alkoxyl group and a hydroxyl group;
n is an integer selected from 1, 2 and 3;
A is a -NH- group or a -C(O)NH- group;
W represents a spacer group selected from -(CH₂)ₘ-, where m is 5 or 6, and the group of formula (II): 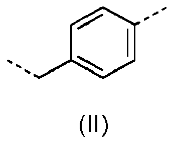
wherein the dashed lines represent the covalent unions with the groups A and - C(=O)-NH-Z;
Z is selected from a hydroxyl group and a group of formula (III): 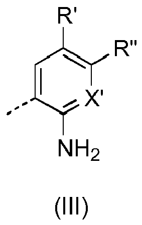 wherein:
the dashed line represents the covalent union with group W-C(=O)-NH-;
X' is selected from a -CH- group and a N atom; and
R' and R" are independently selected from a H atom, an alkyl, a substituted or unsubstituted aryl and a heteroaryl group;
or a solvate or a salt or prodrug thereof.

2. The compound according to claim 1, wherein at least one of X and Y is a -C-R-group, being R selected from hydrogen, an alkyl, an alkoxyl and a hydroxyl group.

3. The compound according to any of claims 1 and 2, wherein both X and Y are a - C-R- group, being R selected from hydrogen, an alkyl, an alkoxyl and a hydroxyl group.

4. The compound according to any one of claims 1 to 3, wherein W is -(CH₂)ₙ-, wherein n is an integer selected from 5 and 6.

5. The compound according to any one of claims 1 to 4, wherein A is -NH-.

6. The compound according to any one of claims 1 to 5, wherein Z is a hydroxyl group.

7. A compound of general formula (I) according to claim 1 selected from the group consisting of:
[1] *N*-Hydroxy-6-[(1,2,3,4-tetrahydroacridin-9-yl)amino]hexanamide, with the following structural formula: 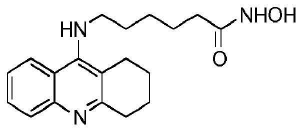
[2] *N*-Hydroxy-7-[(1,2,3,4-tetrahydroacridin-9-yl)amino]heptanamide, with the following structural formula: 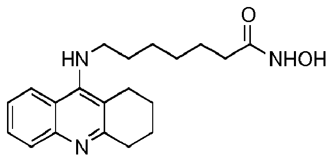
[3] 7-{(2,3-dihydro-1*H*-cyclopenta[*b*]quinolyl)amino}-*N*-hydroxyheptanamide, with the following structural formula: 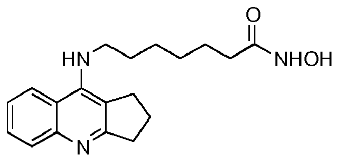
[4] *N*-Hydroxy-7-{(7,8,9,10-tetrahydro-6*H*-cyclohepta[*b*]quinolin-11-yl)amino} heptanamide, with the following structural formula: 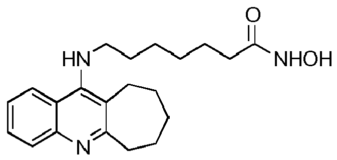
[5] *N*-Hydroxy-7-[(5-methoxy-1,2,3,4-tetrahydroacridin-9-yl)amino]heptanamide, with the following structural formula: 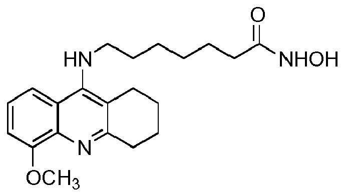
[6] *N*-Hydroxy-7-[(5-hydroxy-1,2,3,4-tetrahydroacridin-9-yl)amino]heptanamide, with the following structural formula: 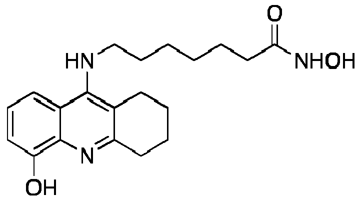
[7] *N*-Hydroxy-7-{(6,7,8,9-tetrahydrobenzo[*b*][1,8]naphthyridin-5-yl)amino} heptanamide, with the following structural formula: 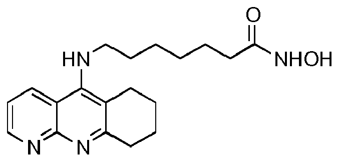
[8] *N*-Hydroxy-7-{(6,7,8,9-tetrahydrobenzo[*b*][1,7]naphthyridin-5-yl)amino} heptanamide, with the following structural formula: 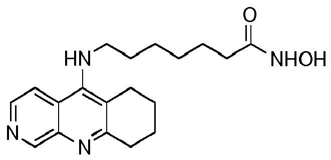
[9] *N*-Hydroxy-4-{[(1,2,3,4-tetrahydroacridin-9-yl)amino]methyl}benzamide, with the following structural formula: 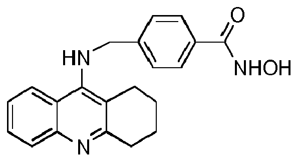
[10] *N*-[6-(hydroxyamino)-6-oxohexyl]-1,2,3,4-tetrahydroacridine-9-carboxamide, with the following structural formula: 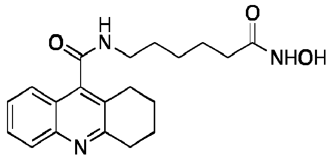
[11] *N*-[7-(hydroxyamino)-7-oxoheptyl]-1,2,3,4-tetrahydroacridine-9-carboxamide, with the following structural formula: 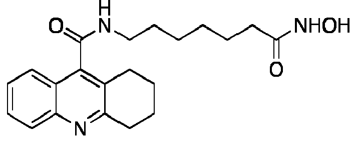
[12] N-(2-Amino-4-methylphenyl)-7-[(1,2,3,4-tetrahydroacridin-9-yl)amino] heptanamide, with the following structural formula: 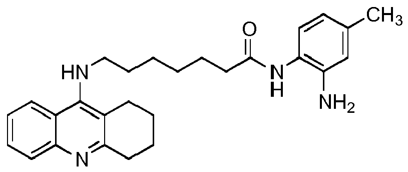
[13] N-(2-Amino-5-methylphenyl)-7-[(1,2,3,4-tetrahydroacridin-9-yl)amino] heptanamide, with the following structural formula: 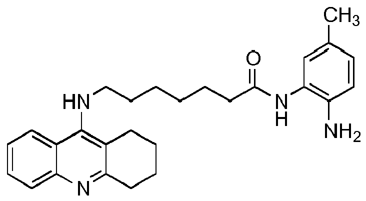
[14] *N*-[2-Amino-5-(*tert*-butyl)phenyl]-7-[(1,2,3,4-tetrahydroacridin-9-yl)amino] heptanamide, with the following structural formula: 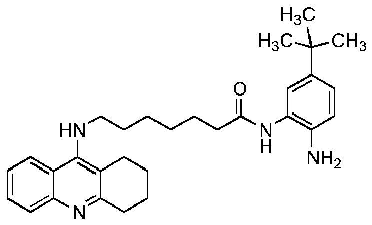
[15] *N*-(4-Amino-[1,1'-biphenyl]-3-yl)-7-[(1,2,3,4-tetrahydroacridin-9-yl)amino] heptanamide, with the following structural formula: 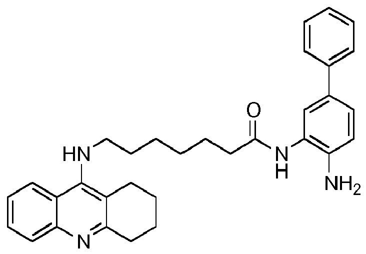
[16] *N*-(4-Amino-[1,1'-biphenyl]-3-yl)-6-[(1,2,3,4-tetrahydroacridin-9-yl)amino] hexanamide, with the following structural formula: 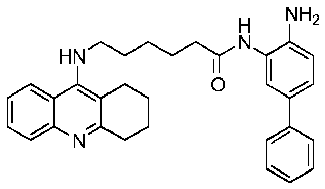
[17] *N*-(4-Amino-3'-methyl-[1,1'-biphenyl]-3-yl)-7-[(1,2,3,4-tetrahydroacridin-9-yl)amino]heptanamide, with the following structural formula: 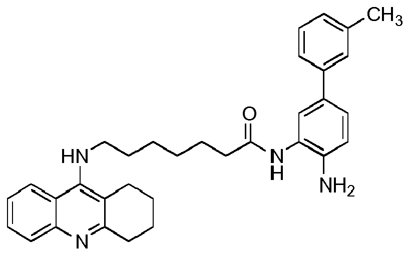
[18] *N*-{4-Amino-4'-(*tert*-butyl-[1,1'-biphenyl]-3-yl}-7-[(1,2,3,4-tetrahydroacridin-9-yl)amino]heptanamide, with the following structural formula: 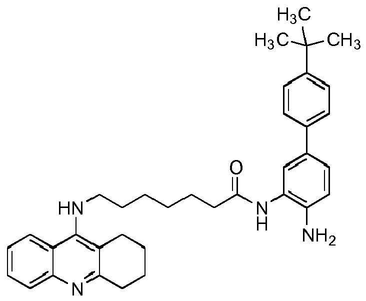
[19] *N*-[2-Amino-5-(naphthalen-2-yl)phenyl]-7-[(1,2,3,4-tetrahydroacridin-9-yl)amino] heptanamide, with the following structural formula: 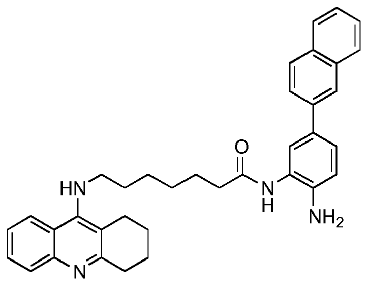
[20] *N*-(2-Amino-5-phenylpyridin-3-yl)-7-[(1,2,3,4-tetrahydroacridin-9-yl)amino] heptanamide, with the following structural formula: 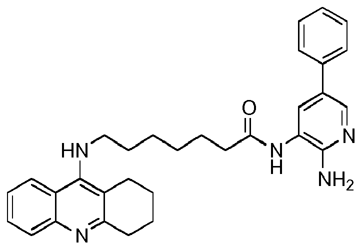
[21] *N*-[2-Amino-5-(pyridin-3-yl)phenyl]-7-[(1,2,3,4-tetrahydroacridin-9-yl)amino] heptanamide, with the following structural formula: 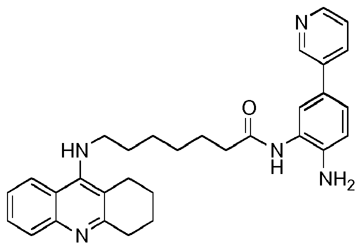
or a solvate or a salt or prodrug thereof.

8. A process for the preparation of a compound of general formula (I) as defined in claim 1, wherein X, Y, W and n have the meaning given in claim 1, A is NH and Z is OH, which comprises:
a) reacting an amino acid of general formula (IV), 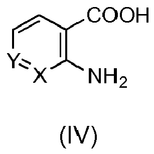 wherein X and Y are independently selected from a N atom or a C-R group, wherein R is selected from a hydrogen atom, an alkyl, an alkoxyl group and a hydroxyl group;
with a cyclic ketone of general formula (V), 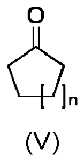 wherein n is selected from 1, 2 and 3;
in the presence of an appropriate chlorination-condensation reagent, to afford a compound of formula (VI): 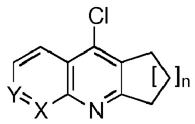 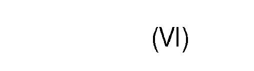 wherein X, Y and n have the meaning given above;
b) reacting the compound of formula (VI) with an ester of general formula X⁻H₃N⁺-W-COOR'", wherein W represents a spacer group selected from -(CH₂)ₘ-, where m is 5 or 6, and the group of formula (II): 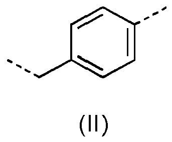 wherein the dashed lines represent the covalent unions with the groups X-H₃N⁺ and -COOR'"; and R'" is a linear or branched alkyl group, and X⁻ is an organic or inorganic anion,
in the presence of an organic base, and an appropriate solvent,
to afford a compound of formula (VII): 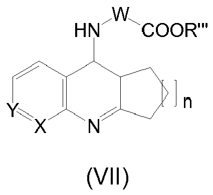 wherein X, Y, n, W and R'" have the meaning given above;
c) reacting the compound of formula (VII) with hydroxylamine hydrochloride, in the presence of a liquid alcohol and a solution of a suitable metallic alkoxide in the previously indicated liquid alcohol and an acid-base indicator.

9. A process for the preparation of a compound of general formula (I) as defined in claim 1, wherein X, Y, W and n have the meaning given in claim 1, A is NH and Z is a group of formula (III) as defined in claim 1, which comprises:
a) reacting an ester of general formula (VII) prepared according to claim 8, 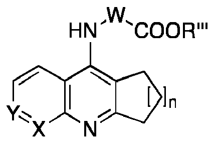 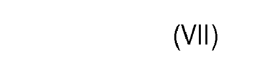 wherein X, Y, n, W and R'" have the meaning given in claim 8,
with an inorganic hydroxide dissolved or suspended in the appropriate volume of water, in the presence of a polar protic solvent,
to afford a compound of formula (VIII): 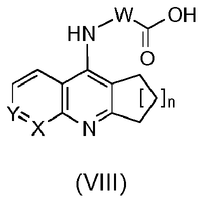 wherein:
X and Y are independently selected from a N atom or a C-R group, wherein R is selected from a hydrogen atom, an alkyl, an alkoxyl group and a hydroxyl group; and
W represents a spacer group selected from -(CH₂)ₘ-, where m is 5 or 6, and the group of formula (II): 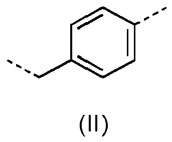 wherein the dashed lines represent the covalent unions with the groups NH and COOH;
b) reacting the compound of formula (VIII) with a protected diamine of general formula (IX): 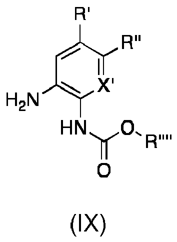 wherein:
X' is selected from a -CH- group and a N atom; and
R' and R" are independently selected from a H atom, an alkyl, a substituted or unsubstituted aryl and a heteroaryl group; and
R"" is a tert-butyl, a benzyl or a 9-fluorenemethyl group,
in the presence of an organic base, a coupling reagent, and an appropriate solvent,
to afford the compound of formula (X): 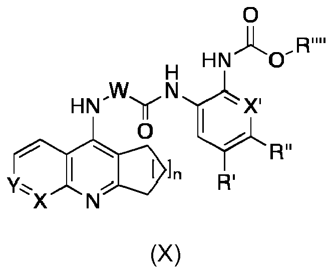
wherein the meaning of X, Y, n, W, X', R', R" and R"" are given above,
c) deprotecting the compound of formula (X) in the presence of an appropriate deprotecting agent and an appropriate solvent.

10. A process for the preparation of a compound of general formula (I) as defined in claim 1, wherein X, Y, W and n have the meaning given in claim 1, A is C(O)NH and Z is OH, which comprises:
a) reacting a compound of formula (XI), 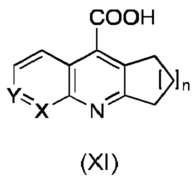 wherein X and Y are independently selected from a N atom or a C-R group, wherein R is selected from a hydrogen atom, an alkyl, an alkoxyl group and a hydroxyl group; and
n is selected from 1, 2 and 3;
with an ester of general formula X⁻H₃N⁺-W-COOR'", wherein W, R'" and X⁻ have the meaning indicated in claim 8, in the presence of an organic base, a coupling reagent, and an appropriate solvent,
to afford a compound of formula (XII): 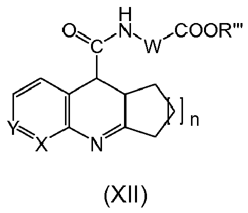 wherein X, Y, n, W and R'" have the meaning given above; and
b) reacting the compound of formula (XII) with hydroxylamine hydrochloride, in the presence of a liquid alcohol, a solution of a suitable metallic alkoxide in the previously indicated liquid alcohol, and an acid-base indicator.

11. A process for the preparation of a compound of general formula (I) as defined in claim 1, wherein X, Y, W and n have the meaning given in claim 1, A is C(O)NH and Z is a group of formula (III) as defined in claim 1, which comprises:
a) reacting an ester of general formula (XII) prepared as defined in claim 10, with an inorganic hydroxide dissolved or suspended in the appropriate volume of water, in the presence of a polar protic solvent, to afford the compound of formula (XIII): 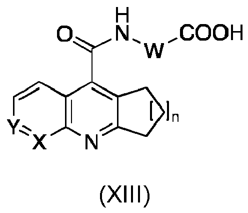 wherein X, Y, n and W have the meaning given in claim 10;
b) reacting a compound of formula (XIII) with a protected diamine of general formula (IX): 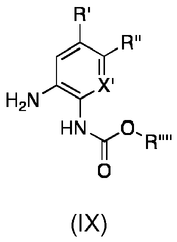 wherein:
X' is selected from a -CH- group and a N atom;
R' and R" are independently selected from a H atom, an alkyl, a substituted or unsubstituted aryl and a heteroaryl group; and
R"" is a tert-butyl, a benzyl or a 9-fluorenemethyl group,
in the presence of an organic base, a coupling reagent, and an appropriate solvent, to afford the compound of formula (XIV): 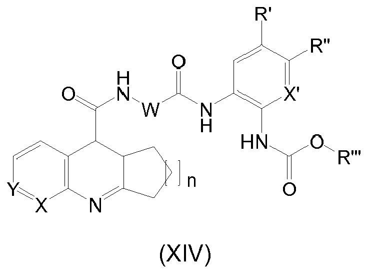 wherein X, Y, n, W, X', R', R" and R"" have the meaning given above;
c) deprotecting the compound of formula (XIV) in the presence of an appropriate deprotecting agent and an appropriate solvent.

12. A compound of formula (I) as defined in any of claims 1 to 7, or a pharmaceutically acceptable solvate or a salt or prodrug thereof, for the use as a medicament.

13. A compound of formula (I) as defined in any of claims 1 to 7, or a pharmaceutically acceptable solvate or a salt or prodrug thereof, for the use in the treatment of a disease or condition selected from the group consisting of cancer, hematological malignancy, proliferative diseases, neurological disorders and immunological disorders.

14. A pharmaceutical composition that comprises at least a compound of formula (I) as defined in any of claims 1 to 7, or a pharmaceutically acceptable solvate or a salt or prodrug thereof, and at least a pharmaceutically acceptable excipient.
